(19)

Europäisches
Patentamt
European
Patent Office
Office européen
des brevets

(11) **EP 4 397 976 A1**

(12) **EUROPEAN PATENT APPLICATION**

(43) Date of publication:
**10.07.2024  Bulletin 2024/28**

(21) Application number: **23150706.2**

(22) Date of filing: **09.01.2023**

(51) International Patent Classification (IPC):
***G01N 33/68*** (2006.01)

(52) Cooperative Patent Classification (CPC):
**G01N 33/6893;** G01N 2333/4716; G01N 2333/78;
G01N 2400/40; G01N 2800/347

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **x-kidney diagnostics GmbH**
**99084 Erfurt (DE)**

(72) Inventors:
• **RHODE, Heidrum**
 **07747 Jena (DE)**
• **TAUTKUS, Bärbel**
 **07747 Jena (DE)**
• **SCHITKE, Julia**
 **07747 Jena (DE)**

• **JOHN-KROEGEL, Ulrike**
 **07747 Jena (DE)**
• **MÜLLER, Katrin**
 **07747 Jena (DE)**
• **WEIGEL, Friederike**
 **07747 Jena (DE)**
• **DOST, Axel**
 **07747 Jena (DE)**
• **KÄMMER, Bettina**
 **07747 Jena (DE)**
• **METZING, Oliver**
 **07747 Jena (DE)**
• **BORNER, Susanne**
 **07747 Jena (DE)**

(74) Representative: **Engelhard, Markus**
 **Boehmert & Boehmert**
 **Anwaltspartnerschaft mbB**
 **Pettenkoferstrasse 22**
 **80336 München (DE)**

(54) **BIOMARKERS FOR THE PRE-CLINICAL AND/OR EARLY-STAGE DETECTION AND/OR
DIAGNOSIS OF KIDNEY DISEASES**

(57)    The present invention relates to biomarkers for the pre-clinical and/or early-stage detection and/or diagnosis of a kidney disease. In particular, a combination of at least two proteins such as selected from Collagen type XIII (COLXIII), Hyaluronan-Binding Protein 2 (HABP2), C4 Binding Protein (C4BP), Complement Factor H (CFH), and Complement Factor I (CFI) is provided for use as biomarker in a method for the pre-clinical and/or early-stage detection and/or diagnosis of a kidney disease. The present invention further relates to a method for the pre-clinical and/or early-stage detection and/or diagnosis of a kidney disease, comprising (a) determining the concentration of at least two proteins in a sample of a subject, and (b) comparing each of the concentrations determined in step (a) with a control value, wherein a deviation of each of the concentrations determined in step (a) from said control value is indicative of a pre-clinical and/or early-stage kidney disease.

EP 4 397 976 A1

**Description**

The present invention relates to biomarkers for the pre-clinical and/or early-stage detection and/or diagnosis of a kidney disease. In particular, a combination of at least two proteins such as selected from Collagen type XIII (COLXIII), Hyaluronan-Binding Protein 2 (HABP2), C4 Binding Protein (C4BP), Complement Factor H (CFH), and Complement Factor I (CFI) is provided for use as biomarker in a method for the pre-clinical and/or early-stage detection and/or diagnosis of a kidney disease. The present invention further relates to a method for the pre-clinical and/or early-stage detection and/or diagnosis of a kidney disease, comprising (a) determining the concentration of at least two proteins in a sample of a subject, and (b) comparing each of the concentrations determined in step (a) with a control value, wherein a deviation of each of the concentrations determined in step (a) from said control value is indicative of a pre-clinical and/or early-stage kidney disease.

BACKGROUND OF THE INVENTION

Nearly all kidney diseases start without any symptoms and pain. Most kidney diseases progress undetectably over several months/years before manifestation and diagnosis. There are no guidelines for the preventive screening of preclinical kidney diseases although these diseases could be stopped or even reversed during this period. The injured glomerular filtration apparatus leads to the leakage of plasma proteins into the primary urine. Currently, proteinuria or albuminuria is (besides hematuria) the earliest parameter indicating kidney injury. However, this parameter indicates an already manifested disease and not an early stage thereof. Moreover, albuminuria triggers further progression of kidney destruction themselves. Clinically established parameters for kidney function, like creatinine in serum, show pathological values much too late, when already 50% of kidney function is disturbed. Thus, there is a still unresolved diagnostic dilemma.

Practically all kidney diseases progressing to end stage kidney disease (ESKD) requiring kidney replacement therapy would be treatable and even curable when detected and treated in a time close to onset. If nephropathies would be detectable pre-emptively before measurable albuminuria so there would be therapeutic options to slow down, stop or even reverse their progress.

For example, the Alport syndrome (AS) is a hereditary nephropathy characterized by hematuria and albuminuria, often leading to ESKD, hearing loss, and ocular changes (see e.g., Hudson *et al.,* 2003; Nagel *et al.,* 2005). The majority of cases is X-linked inherited due to mutations in the $\alpha$5 chain of collagen (IV) (COL(IV)A5) (Hertz *et al.,* 2012). Female carriers of X-linked AS also may be seriously affected (Goka *et al.,* 2021). Moreover, there is a considerable genetic and phenotypic variability (Savige *et al.,* 2018). Some alterations have been pre-clinically identified in the renal cortex of affected AS-mice (Gessel *et al.,* 2019; Dufek *et al.,* 2016). Despite this, the entire chain of specific pathogenic events in AS that lead from a type IV collagen mutation to progressive kidney fibrosis remains unclear. Every child is born healthy with immature $\alpha$1/2(IV)-chains, but - due to the mutation - AS patients are unable to mature their GBM with $\alpha$3/4/5(IV)-chains. Therefore, the defect manifests sometimes after birth with a "window of opportunity" for diagnostics. Recent expert guidelines recommend genetic testing for the diagnosis of Alport syndrome as well as for the identification of some modifiers (Savige *et al.,* 2019). Nevertheless, the application of these methods usually requires anamnestic and/or clinical evidence like hematuria (Gross *et al.,* 2020). However, in previously undiagnosed families and with de-novo mutations of AS, this tool is unfeasible for early diagnostics. Histological analysis is highly invasive, risky, and depicts rather late events of progression. To date transplantation is the only therapy to cure (Savige *et al.,* 2019) of ESKD due to AS. Despite this, currently, the most effective and widely used therapy is albeit, the inhibition of the renin-angiotensin-aldosterone system. In addition, several new approaches are under investigation to improve kidney functionality and prolong life expectancy. Such approaches include the use of chaperones, stem cells, stem cell extracellular vesicle, and exon skipping (see e.g., Yamamura *et al.,* 2020). Currently, ACE-inhibition has been shown to delay kidney failure by many years in a time-dependent manner (Kashtan *et al.,* 2013; Noone *et al.,* 2013). That is, the earlier treatment commences, the greater the benefit. Generally, the start of therapy is most benefit to affected children who are not yet symptomatic and optimally delay progression of their kidney disease.

Hence, early progressive biomarkers indicative of glomerular alarm signals before any clinical sign of glomerular injury are urgently required for all, to ensure early diagnosis, to early start of therapy, to monitor therapy, as well as to develop new therapeutic strategies.

In addition, in developed countries the diabetic nephropathy (DN) is the leading cause of end-stage kidney diseases (ESKDs) and kidney replacement therapy. However, diagnostic tests established in clinical practice, i.e. serum creatinine, estimated glomerular filtration rate (eGFR) and albuminuria indicate rater late and irreversible stages of DN. In the scientific literature there are no practicable biomarker in routine use that could indicate a beginning DN in a pre-clinical and curable state before onset of (micro-) albuminuria. Moreover, there are also no indicators to differentiate diabetic individuals more susceptible to nephropathy and kidney failure from others. DN is initiated by alterations of endothelia and basement membrane of the kidney glomerulus. The same holds true for several other glomerulo-nephropathies. Moreover, according to scientific literature there is tiny congruence of the early molecular alterations and histo-pathogenesis of most glomerulopathies irrespective of their cause (see e.g. Hastings *et al.,* 2021; Koopman *et al.,* 2020), like deposition and activation of complement components, deposits of other material, basement thickening, and podocyte effacement. Uniformly the filtration apparatus including podocytes, endothelia, and mesangial cells are injured like under AS. Not until then these early processes are accessible by the established diagnostics, as discussed above. Later these alterations proceed to the loss of the filtration barrier, albuminuria, tubular alterations, and fibrosis.

SUMMARY OF THE INVENTION

According to the present invention this object is solved by a *combination of at least two proteins* selected from

Collagen type XIII (COLXIII),
Hyaluronan-Binding Protein 2 (HABP2),
C4 Binding Protein (C4BP),
Complement Factor H (CFH), and
Complement Factor I (CFI)

for use as biomarker in a method for the pre-clinical and/or early-stage detection and/or diagnosis of a kidney disease in a mammal.

According to the present invention this object is solved by *a protein* selected from

Collagen type XIII (COLXIII),
Hyaluronan-Binding Protein 2 (HABP2),
C4 Binding Protein (C4BP),
Complement Factor H (CFH), and
Complement Factor I (CFI)

*in combination with at least one further protein* which is selected from:

Fibrinogen,
Lumican (LUM),
Formin 1 (FMN),
Vitronectin (VTN),
Gelsolin (GS),
Angiotensinogen (AGT),
Adiponectin (ADP),
Transforming growth factor-$\beta$1 (TGF-$\beta$1),
Carboxyterminal Propeptid of Type I Procollagen (PICP),
C Reactive Protein (CRP),
$\alpha$-1-Acid Glycoprotein (a1AGP),
Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich $\alpha$-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK),
Myosin IXA (MYIXA),
Kininogen 1,
$\alpha$2-HS Glycoprotein,
Complement C6,

Complement C7,
Complement C8,
APOH, Beta-2-Glycoprotein 1,
Serpin family A member 1,
Transferrin,
α1-B Glycoprotein,
Apo AI,
Apo D,
Alpha-1-microglobulin/bikunin precursor,
Carboxypeptidase N subunit 2, and
Serine/arginine repetitive matrix protein 3,

for use as biomarker in a method for the pre-clinical and/or early-stage detection and/or diagnosis of a kidney disease in a mammal.

According to the present invention this object is solved by a *combination of at least two proteins* selected from

Collagen type XIII (COLXIII),
Hyaluronan-Binding Protein 2 (HABP2),
C4 Binding Protein (C4BP),
Complement Factor H (CFH),
Complement Factor I (CFI),
Fibrinogen,
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP),
C Reactive Protein (CRP),
α-1-Acid Glycoprotein (a1AGP),
Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK),
Myosin IXA (MYIXA),
Kininogen 1,
α2-HS Glycoprotein,
Complement C6,
Complement C7,
Complement C8,
APOH, Beta-2-glycoprotein 1,
Serpin family A member 1,
Transferrin,
α1-B Glycoprotein,
Apo AI,
Apo D,

Alpha-1-microglobulin/bikunin precursor,
Carboxypeptidase N subunit 2,
Serine/arginine repetitive matrix protein 3
Complement C1s,
Complement C1r,
Complement C2,
Complement C3,
Complement C4,
Complement C5,
Complement factor B,
Haptoglobin,
$\alpha$2-macroglobulin,
Alpha-1-antitrypsin,
Ceruloplasmin,
Serpin family A member 3,
Serpin family A member 5,
Serpin family A member 7,
Serpin family G member 1,
Serpin family D member 1,
Coagulation factor V,
Prothrombin,
Plasminogen,
Protein S,
Afamin,
Transthyretin,
Hemoglobin subunit alpha,
Fetuin B,
Insulin like growth factor binding protein,
Alpha-2-glycoprotein 1,
Paraoxonase 1,
Dipeptidyl peptidase 4,
C-type lectin domain family 3 member B,
Galectin 3 binding protein,
Apolipoprotein AIV (Apo AIV),
Apolipoprotein CI (Apo CI),
Apolipoprotein CII (Apo CII),
Apolipoprotein E (Apo E4),
Apolipoprotein M (Apo M),
Apolipoprotein B (Apo B),
Myosin IB,
Myosin XVIIIB,
Kinesin-like protein,
CD109 molecule,
Maltase-glucoamylase,
Sacsin molecular chaperone,
Dispatched RND transporter family member 2,
DNA Polymerase,
Trafficking protein particle complex subunit 2,
RB associated KRAB zinc finger,
Ciliogenesis-associated TTC17-interacting protein,
Proteasome subunit alpha type,
Regulating synaptic membrane exocytosis 2,
Extracellular matrix protein 1,
Cadherin 5,
Coiled-coil domain containing 178, and
Attractin,

for use as biomarker in a method for the pre-clinical and/or early-stage detection and/or diagnosis of a kidney disease in a mammal.

According to the present invention this object is solved by a *method for the pre-clinical and/or early-stage detection and/or diagnosis of a kidney disease,* comprising

(a) determining the concentration of at least two proteins in a sample of a mammal,
wherein said at least two proteins are selected from

Collagen type XIII (COLXIII),
Hyaluronan-Binding Protein 2 (HABP2),
C4 Binding Protein (C4BP),
Complement Factor H (CFH), and
Complement Factor I (CFI),

or

wherein said at least two proteins are selected from
one protein which selected from

Collagen type XIII (COLXIII),
Hyaluronan-Binding Protein 2 (HABP2),
C4 Binding Protein (C4BP),
Complement Factor H (CFH), and
Complement Factor I (CFI),

and at least one further protein which is selected from

Fibrinogen,
Lumican (LUM),
Formin 1 (FMN),
Vitronectin (VTN),
Gelsolin (GS),
Angiotensinogen (AGT),
Adiponectin (ADP),
Transforming growth factor-$\beta$1 (TGF-$\beta$1),
Carboxyterminal Propeptid of Type I Procollagen (PICP),
C Reactive Protein (CRP),
$\alpha$-1-Acid Glycoprotein (a1AGP),
Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich $\alpha$-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK),
Myosin IXA (MYIXA),
Kininogen 1,
$\alpha$2-HS Glycoprotein,
Complement C6,
Complement C7,
Complement C8,
APOH, Beta-2-glycoprotein 1,

Serpin family A member 1,
Transferrin,
α1-B Glycoprotein,
Apo AI,
Apo D,
Alpha-1-microglobulin/bikunin precursor,
Carboxypeptidase N subunit 2, and
Serine/arginine repetitive matrix protein 3,

(b) comparing each of the concentrations determined in step (a) with a control value,

wherein a deviation of each of the concentrations determined in step (a) from said control value is indicative of a pre-clinical and/or early-stage kidney disease in said mammal.

According to the present invention this object is solved by a *method for the pre-clinical and/or early-stage detection and/or diagnosis of a kidney disease,* comprising

(a) determining the concentration of at least two proteins in a sample of a mammal, wherein said at least two proteins are selected from

Collagen type XIII (COLXIII),
Hyaluronan-Binding Protein 2 (HABP2),
C4 Binding Protein (C4BP),
Complement Factor H (CFH),
Complement Factor I (CFI),
Fibrinogen,
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP),
C Reactive Protein (CRP),
α-1-Acid Glycoprotein (a1AGP),
Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK),
Myosin IXA (MYIXA),
Kininogen 1,
α2-HS Glycoprotein,
Complement C6,
Complement C7,
Complement C8,
APOH, Beta-2-glycoprotein 1,
Serpin family A member 1,
Transferrin,

α1-B Glycoprotein,
Apo AI,
Apo D,
Alpha-1-microglobulin/bikunin precursor,
Carboxypeptidase N subunit 2,
Serine/arginine repetitive matrix protein 3,
Complement C1s,
Complement C1r,
Complement C2,
Complement C3,
Complement C4,
Complement C5,
Complement factor B,
Haptoglobin,
α2-macroglobulin,
Alpha-1-antitrypsin,
Ceruloplasmin,
Serpin family A member 3,
Serpin family A member 5,
Serpin family A member 7,
Serpin family G member 1,
Serpin family D member 1,
Coagulation factor V,
Prothrombin,
Plasminogen,
Protein S,
Afamin,
Transthyretin,
Hemoglobin subunit alpha,
Fetuin B,
Insulin like growth factor binding protein,
Alpha-2-glycoprotein 1,
Paraoxonase 1,
Dipeptidyl peptidase 4,
C-type lectin domain family 3 member B,
Galectin 3 binding protein,
Apolipoprotein AIV (Apo AIV),
Apolipoprotein CI (Apo CI),
Apolipoprotein CII (Apo CII),
Apolipoprotein E (Apo E4),
Apolipoprotein M (Apo M),
Apolipoprotein B (Apo B),
Myosin IB,
Myosin XVIIIB,
Kinesin-like protein,
CD109 molecule,
Maltase-glucoamylase,
Sacsin molecular chaperone,
Dispatched RND transporter family member 2,
DNA Polymerase,
Trafficking protein particle complex subunit 2,
RB associated KRAB zinc finger,
Ciliogenesis-associated TTC17-interacting protein,
Proteasome subunit alpha type,
Regulating synaptic membrane exocytosis 2,
Extracellular matrix protein 1,
Cadherin 5,
Coiled-coil domain containing 178, and

Attractin,

(b) comparing each of the concentrations determined in step (a) with a control value,

wherein a deviation of each of the concentrations determined in step (a) from said control value is indicative of a pre-clinical and/or early-stage kidney disease in said mammal.

## DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 90%". Furthermore, such an interpretation should apply regardless of the breadth of the range or the characteristics being described.

*Pre-clinical and early-stage biomarkers for kidney diseases*

As outlined above, the present invention provides novel biomarkers for the pre-clinical and/or early-stage detection and/or diagnosis of a kidney disease.

The biomarkers of the present invention are the proteins disclosed herein. The term "protein", as used herein, also refers to / comprises modified variants of the respective protein, fragments of the respective protein, protein chains and complexes of the respective protein with further proteins.

In addition, the term "protein", as used herein, refers to / comprises all known proteins enclosing the identified chains of the respective protein, modified variants of the respective protein, fragments of the respective protein, single protein chains, multiple protein chains and complexes of the respective protein (chain) with further proteins and protein chains, irrespective of their validation using a currently available immune assay like ELISA.

There are stages defined describing the course of chronic kidney diseases (CKD) of variable origins (see Jerums *et al.,* 2009):

| Stage I: | hyperfiltration; |
| --- | --- |
| Stage II: | impairment with normoalbuminuria; |
| Stage III: | microalbuminuria; |
| Stage IV: | macroalbuminuria; |
| Stage V: | end-stage kidney failure. |

In Alport syndrome (AS) the following stages are described for kidney damage and loss of function, stage definition according Gross *et al.* (2012):

| Stage 0: | microhaematuria without microalbuminuria (usually at birth), |
| --- | --- |
| Stage I: | microalbuminuria (30-300 mg albumin/gCrea), |
| Stage II: | albuminuria >300 mg albumin/gCrea, |
| Stage III: | >25% decline of normal kidney function (creatinine clearance), |
| Stage IV: | end-stage kidney failure. |

Diabetic kidney disease (DKD) is staged according to albuminuria and additionally considering eGFR as follows (see de Boer *et al.,* 2020):

eGFR (ml/min/1.73m$^2$);
Stage G1: ≥90;
Stage G2: 60-89
Stage G3a: 45-59
Stage G3b: 30-44
Stage G4: 15-29
Stage G5: <15
Albuminuria (mg/g creatinine)
Stage A1: <30
Stage A2: 30-300
Stage A3: >300...

In the present invention, stages I and II (as defined for CKD of variable origins), stages G1A1 G2A2, G3aA1 (as defined for DKD) and stages 0 and I (as defined for AS), are addressed by the biomarkers. There is a lack of any currently accepted, applicable and specific clinical and laboratory sign to diagnose the "(beginning) kidney failure". During these stages, the disease is ongoing but silent. Contrarily, in literature, the term "early" mostly refers to early stages of manifested kidney impairment already detectable by various commonly applied biomarkers such as e.g. KIM-1, NGAL, cystatin C, and microalbuminuria.

A "pre-clinical" stage of a kidney disease as used herein refers to stage I (hyperfiltration) as defined for CKD of variable origins and to stage 0 (microhaematuria without microalbuminuria) as defined for AS.

An "early" stage of a kidney disease as used herein refers to stage II (impairment with normoalbuminuria) as defined for CKD of variable origins, to stage G1A2, G2A2, G3aA1 as defined for DKD and stage I (microalbuminuria (30-300 mg albumin/g creatinine) as defined for AS.

The present invention provides the biomarkers for detecting and/or diagnosing a kidney disease in its pre-clinical and/or early stage. In other words, due to the biomarkers it is possible to detect that a kidney disease is in its pre-clinical and/or early stage, which allows the diagnosis for a subject/patient that he or she is developing a kidney disease, which is at the time of taking the sample in its pre-clinical and/or early stage.

Preferably, the kidney disease is selected from chronic progressive kidney diseases

starting with glomerular injury (glomerulopathy),
preferably post-infectious glomerulopathy, IgA-nephropathy, Henoch-Schoenlein-Purpura, Focal segmental glomerulosclerosis, Alport syndrome, thin basement membrane nephropathy, benign familiar hematuria,
or
starting on other parts of the body, with other dysregulations, and/or other parts of the
kidney with implication on the glomerulus (nephropathy),
preferably metabolic syndrome, hypertension, heart failure, nephrotoxic medication, adipositas, diabetes mellitus (diabetic nephropathy), renal agenesis, renal hypoplasia, multicystic renal dysplasia, autosomal dominant polycystic disease, autosomal recessive polycystic disease, anomalies of the urinary tract, duplex kidneys, nephronophthisis.

The present invention provides a *combination of at least two proteins* selected from

Collagen type XIII (COLXIII),
Hyaluronan-Binding Protein 2 (HABP2),
C4 Binding Protein (C4BP), preferably C4BP alpha chain (C4BPA),
Complement Factor H (CFH), and
Complement Factor I (CFI)

for use as biomarker in a method for the pre-clinical and/or early-stage detection and/or diagnosis of a kidney disease.

By the term "combination" as used herein it is only meant that at least two biomarkers are used for detecting and/or diagnosing. One biomarker is used together with at least one further biomarker for the detection and/or diagnosis.

In one embodiment, 2, 3, 4, 5, 6, 7, 8, 9, 10, 11, 12, 13, 14, 15, 16, 17, 18, 19, 20, or even further biomarkers are used.

The present invention provides *at least two proteins* selected from

Collagen type XIII (COLXIII),

Hyaluronan-Binding Protein 2 (HABP2),
C4 Binding Protein (C4BP), preferably C4BP alpha chain (C4BPA),
Complement Factor H (CFH), and
Complement Factor I (CFI)

for use as biomarker in a method for the pre-clinical and/or early-stage detection and/or diagnosis of a kidney disease.

In a preferred embodiment, COLXIII and C4BP, or COLXIII and C4BP and HABP2, or CFH and CFI are determined.

In one embodiment, COLXIII, HABP2, C4BP, CFH, and CFI are determined.

The present invention provides a *protein* selected from

Collagen type XIII (COLXIII),
Hyaluronan-Binding Protein 2 (HABP2),
C4 Binding Protein (C4BP), preferably C4BP alpha chain (C4BPA),
Complement Factor H (CFH), and
Complement Factor I (CFI)

*in combination with at least one further protein* which is selected from:

Fibrinogen, preferably Fibrinogen γ-chain (FGG),
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP), and
C Reactive Protein (CRP),
α-1-Acid Glycoprotein (a1AGP),
Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN), preferably dynein heavy chain domain 1,
Fukutin (FUK),
Myosin IXA (MYIXA),
Kininogen 1,
α2-HS Glycoprotein,
Complement C6,
Complement C7,
Complement C8, preferably C8 alpha chain and/or C8 beta chain,
APOH, Beta-2-glycoprotein 1,
Serpin family A member 1,
Transferrin,
α1-B Glycoprotein,
Apo AI,
Apo D,
Alpha-1-microglobulin/bikunin precursor,
Carboxypeptidase N subunit 2,

Serine/arginine repetitive matrix protein 3,
Complement C1s,
Complement C1r,
Complement C2,
Complement C3,
Complement C4,
Complement C5,
Complement factor B,
Haptoglobin,
$\alpha$2-macroglobulin,
Alpha-1-antitrypsin,
Ceruloplasmin,
Serpin family A member 3,
Serpin family A member 5,
Serpin family A member 7,
Serpin family G member 1,
Serpin family D member 1,
Coagulation factor V,
Prothrombin,
Plasminogen,
Protein S,
Afamin,
Transthyretin,
Hemoglobin subunit alpha,
Fetuin B,
Insulin like growth factor binding protein,
Alpha-2-glycoprotein 1,
Paraoxonase 1,
Dipeptidyl peptidase 4,
C-type lectin domain family 3 member B,
Galectin 3 binding protein,
Apolipoprotein AIV (Apo AIV),
Apolipoprotein CI (Apo CI),
Apolipoprotein CII (Apo CII),
Apolipoprotein E (Apo E4),
Apolipoprotein M (Apo M),
Apolipoprotein B (Apo B),
Myosin IB,
Myosin XVIIIB,
Kinesin-like protein,
CD109 molecule,
Maltase-glucoamylase,
Sacsin molecular chaperone,
Dispatched RND transporter family member 2,
DNA Polymerase,
Trafficking protein particle complex subunit 2,
RB associated KRAB zinc finger,
Ciliogenesis-associated TTC17-interacting protein,
Proteasome subunit alpha type,
Regulating synaptic membrane exocytosis 2,
Extracellular matrix protein 1,
Cadherin 5,
Coiled-coil domain containing 178, and
Attractin,

for use as biomarker in a method for the pre-clinical and/or early-stage detection and/or diagnosis of a kidney disease.

The protein biomarkers of the present invention are shown in Table 1, where also the protein IDs and the deviation of the concentration determined by mass spectrometry (mean ratio in sub-fractions or overall trend) in affected dogs in comparison to healthy controls are shown. The protein IDs refer to the UniProt database of ~47,800 canine entries (date: October 26, 2017). Practically, all identified proteins possess a predominant origin usually known for a long time, but are also produced by several other organs and tissues, particularly in the kidney. Besides from the liver, several acute phase proteins, including complement components, are produced widespread by various cell types, such as mesangial cells and other kidney cells.

In urine from humans with kidney diseases the following proteins show a deviation of the concentration which is higher than or equal to (≥) 2 times the median of the standard concentration range, determined by the mean ratio (mean ratio in sub-fractions or overall trend, mass spectrometry) as well as by ELISA:

**Collagen type XIII (COLXIII)** refers to a protein with protein ID F1PJH3. Type XIII Collagen is a homotrimer, or a protein composed of three identical peptide chains (monomers), each called an alpha 1 chain of type XIII collagen. Formally, the monomers are called collagen type XIII, alpha-1 chain and in humans are encoded by the *COL3A1* gene. Type XIII collagen is one of the fibrillar collagens whose proteins have a long, inflexible, triple-helical domain. COLXIII is a transmembrane protein expressed in glomerular endothelial cells.

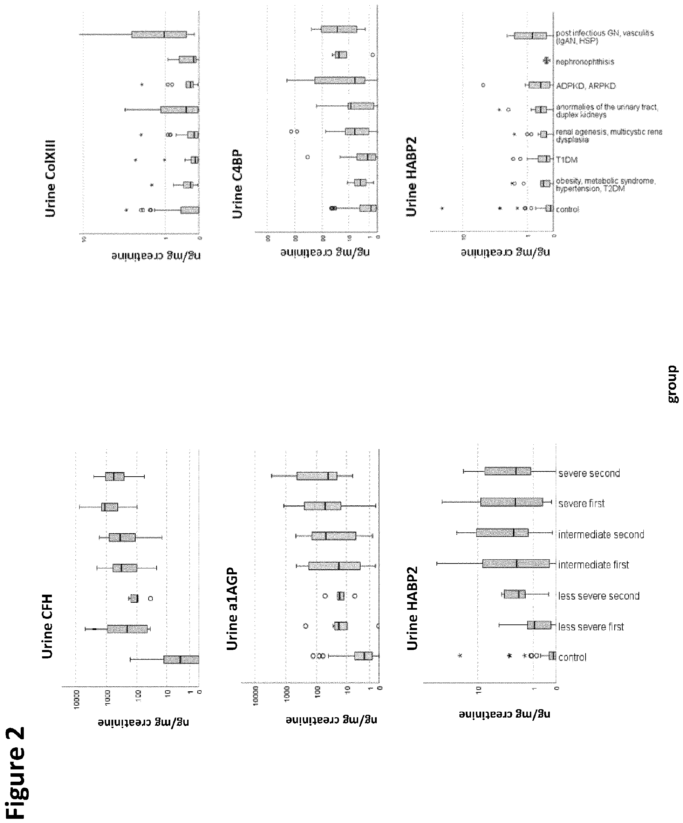

In urine from humans with kidney diseases COLXIII shows a deviation of the concentration which is higher than or equal to (≥) 2 times the median of the standard concentration range, determined by ELISA. See also Figure 2.

**Hyaluronan-Binding Protein 2 (HABP2)** refers to a protein with protein ID J9NV47. Hyaluronan-binding protein 2 also known as factor VII activating protease (FSAP) is a protein that in humans is encoded by the HABP2 gene. The protein encoded by this gene is an extracellular serine protease which binds hyaluronic acid. It is involved in extracellular matrix organization and cell adhesion.

In urine from humans with kidney diseases HABP2 shows a deviation of the concentration which is higher than or equal to (≥) 2 times the median of the standard concentration range, determined by ELISA. See also Figure 2.

**C4 Binding Protein (C4BP)** or complement component 4 binding protein refers to a protein with protein ID F1PGM9. C4b-binding protein is involved in regulation of the complement system. It is a multimeric protein comprising 7 identical alpha chains, encoded by C4BPA, and a single beta chain, encoded by C4BPB. In the kidney it is produced by podocytes. The biomarker is C4BP, preferably C4BP alpha chain (C4BPA).

In urine from humans with kidney diseases C4BPA shows a deviation of the concentration which is higher than or equal to (≥) 2 times the median of the standard concentration range, determined by ELISA. See also Figure 2.

**Complement Factor H (CFH)** refers to a protein with protein ID F1PY40. Factor H is a member of the regulators of complement activation family and is a complement control protein. It is a soluble glycoprotein that circulates in human plasma.

In urine from humans with kidney diseases CFH shows a deviation of the concentration which is higher than or equal to (≥) 2 times the median of the standard concentration range, determined by ELISA. See also Figure 2.

**Complement Factor I (CFI)** refers to a protein with protein ID J9NT17. Complement factor I, also known as C3b/C4b inactivator, is a protein that in humans is encoded by the *CFI* gene. Complement factor I (factor I) is a protein of the complement system that regulates complement activation by cleaving cell-bound or fluid phase C3b and C4b.

In urine from humans with kidney diseases CFI shows a deviation of the concentration which is higher than or equal to (≥) 2 times the median of the standard concentration range, determined by ELISA.

**Fibrinogen γ-Chain (FGG)** refers to a protein with protein ID F 1P8G0. Fibrinogen gamma chain, encoded by the fibrinogen gamma gene (FGG), is a human gene found on chromosome 4. The protein encoded by this gene is the gamma component of fibrinogen, a blood-borne glycoprotein composed of three pairs of nonidentical polypeptide chains. Fibrinogen is also a constituent of the glomerular basement membrane. The biomarker is **fibrinogen,** preferably fibrinogen γ-chain (FGG).

In urine from humans with kidney diseases FGG shows a deviation of the concentration which is higher than or equal to (≥) 2 times the median of the standard concentration range, determined by ELISA.

**Lumican (LUM)** refers to a protein with protein ID E2R416. Lumican, also known as LUM, is an extracellular matrix protein that, in humans, is encoded by the LUM gene on chromosome 12.

In urine from humans with kidney diseases LUM shows a deviation of the concentration which is higher than or equal to (≥) 2 times the median of the standard concentration range, determined by ELISA.

**Formin 1 (FMN)** refers to a protein with protein ID J9P216. Formins (formin homology proteins) are a group of proteins that are involved in the polymerization of actin and associate with the fast-growing end (barbed end) of actin filaments.

In urine from humans with kidney diseases FMN shows a deviation of the concentration which is higher than or equal to (≥) 2 times the median of the standard concentration range, determined by ELISA.

**Vitronectin (VTN)** refers to a protein with protein ID Q2YF02. Vitronectin is a glycoprotein of the hemopexin family which is abundantly found in serum, the extracellular matrix of several tissues including the glomerular basement membrane, and bone. In humans it is encoded by the *VTN* gene. In Table 1, it is referred to as vitronectin (fragment).

In urine from humans with kidney diseases VTN shows a deviation of the concentration which is higher than or equal to (≥) 2 times the median of the standard concentration range, determined by ELISA.

**Gelsolin (GS)** refers to a protein with protein ID F6Y3P9. Gelsolin is an actin-binding protein that is a key regulator of actin filament assembly and disassembly.

In urine from humans with kidney diseases GS shows a deviation of the concentration which is higher than or equal to (≥) 2 times the median of the standard concentration range, determined by ELISA.

**Angiotensinogen (AGT)** refers to a protein with protein ID F1PAL5. Angiotensinogen is a component of the renin-angiotensin system (RAS), a hormone system that regulates blood pressure and fluid balance.

In urine from humans with kidney diseases AGT shows a deviation of the concentration which is higher than or equal to (≥) 2 times the median of the standard concentration range, determined by ELISA.

**Adiponectin (ADP)** refers to a protein with protein ID B5U1S6. Adiponectin (also referred to as GBP-28, apM1, AdipoQ and Acrp30) is a protein hormone and adipokine, which is involved in regulating glucose levels as well as fatty acid breakdown. In humans it is encoded by the *ADIPOQ* gene and it is produced in primarily in adipose tissue, but also in muscle. In Table 1, it is referred to as adiponectin (fragment).

In urine from humans with kidney diseases ADP shows a deviation of the concentration which is higher than or equal to (≥) 2 times the median of the standard concentration range, determined by ELISA.

**Transforming growth factor-β1 (TGF-β1)** refers to a protein with protein ID P54831. Transforming growth factor beta 1 or TGF-β1 is a polypeptide member of the transforming growth factor beta superfamily of cytokines. It is a secreted protein that performs many cellular functions, including the control of cell growth, cell proliferation, cell differentiation, matrix remodeling, and apoptosis. In humans, TGF-β1 is encoded by the *TGFB1* gene. TGF-β1 was not identified by mass spectrometry due to its very low concentration in our sub-fractions (see 1.3.). Since we identified many matrix proteins indicating matrix remodeling, we measured TGF-β1 in human samples.

In urine from humans with kidney diseases TGF-β1 shows a deviation of the concentration which is higher than or equal to (≥) 2 times the median of the standard concentration range, determined by ELISA.

**Carboxyterminal Propeptid of Type I Procollagen (PICP)** refers to a protein fragment. Procollagen type I carboxy-terminal propeptide (PICP), derived from type I procollagen, has been identified as an indicator of type I collagen synthesis in bone matrix formation and skin recovery. PICP is a fragment of the trimer of collagen I, shown as Collagen α1(I) chain (ID F1Q3I5), Collagen α1 (I) (fragment) (ID E5G723), und Collagen α2 (I) chain (ID F1PHY1) (as listed in Table 1).

In urine from humans with kidney diseases PICP shows a deviation of the concentration which is higher than or equal to (≥) 2 times the median of the standard concentration range, determined by ELISA.

**C Reactive Protein (CRP)** or pentraxin refers to a protein with protein ID T2KEN6. C-reactive protein (CRP) is an annular (ring-shaped) pentameric protein found in blood plasma, whose circulating concentrations rise in response to inflammation. It is an acute-phase protein of hepatic origin that increases following interleukin-6 secretion by macrophages and T cells. Its physiological role is to bind to lysophosphatidylcholine expressed on the surface of dead or dying cells (and some types of bacteria) in order to activate the complement system via C1q. In urine from humans with kidney diseases CRP shows a deviation of the concentration which is higher than or equal to (≥) 2 times the median of the standard concentration range, determined by ELISA.

**α-1-Acid Glycoprotein (a1AGP)** refers to a protein with protein ID F6Y713. Alpha-1-acid glycoprotein (*α₁AGp, AGP* or *AAG*) or orosomucoid (ORM) is an acute phase protein found in plasma. It is an alpha-globulin glycoprotein and is modulated by two polymorphic genes. It is synthesized primarily in hepatocytes.

In urine from humans with kidney diseases a1AGP shows a deviation of the concentration which is higher than or equal to (≥) 2 times the median of the standard concentration range, determined by ELISA. See also Figure 2.

**Complement Component 1q (C1q)** consisting of Complement C1q A chain, Complement C1q B chain, and Complement C1q C chain, refers to a protein with protein ID J9P4B4 (for the A chain), J9P1G2 (for the B chain) and E2RJC2 (for the C chain) (as listed in Table 1). The complement component 1q (C1q) is a protein complex involved in the complement system. C1q together with C1r and C1s form the C1 complex. C1q is composed of 18 polypeptide chains: six A-chains, six B-chains, and six C-chains.

In urine from humans with kidney diseases C1q shows a deviation of the concentration which is higher than or equal to (≥) 2 times the median of the standard concentration range, determined by ELISA.

**Complement Component 9 (C9)** or complement C9 refers to a protein with protein ID J9P8Z6. Complement component 9 (C9) is a MACPF protein involved in the complement system. Once activated, about 12-18 molecules of C9 polymerize to form pores in target cell membranes, causing lysis and cell death. C9 is one member of the complement membrane attack complex (MAC), which also includes complement components C5b, C6, C7 and C8.

In urine from humans with kidney diseases C9 shows a deviation of the concentration which is higher than or equal to (≥) 2 times the median of the standard concentration range, determined by ELISA.

**Leucine Rich α-2-Glycoprotein 1 (LRGP1)** refers to a protein with protein ID E2R833. Leucine-rich alpha-2-glycoprotein 1 is a protein which in humans is encoded by the gene *LRG1*. The leucine-rich repeat (LRR) family of proteins, including LRG1, have been shown to be involved in protein-protein interaction, signal transduction, and cell adhesion and development. LRG1 is expressed during granulocyte differentiation.

In urine from humans with kidney diseases LRGP1 shows a deviation of the concentration which is higher than or equal to (≥) 2 times the median of the standard concentration range, determined by ELISA.

The following biomarkers show a deviation of the concentration at least in dogs with kidney diseases, wherein said deviation is higher than or equal to (≥) 2 times the median of the standard concentration range, determined by mass spectrometry:

**Coagulation factor XIII (CFXIII)** consisting of Coagulation factor XIII A chain and Coagulation factor XIII B chain refers to proteins with protein ID F1PKX3 (for the A chain) and F1Q041 (for the B chain) (as listed in Table 1). Factor XIII or fibrin stabilizing factor possesses also extracellular matrix stabilizing activities, is a zymogen found in blood of humans and some other animals. It is activated by thrombin to factor XIIIa. Factor XIII a is an enzyme of the blood coagulation system that crosslinks fibrin. Deficiency of XIII worsens clot stability and increases bleeding tendency. Human CFXIII is a heterotetramer. It consists of 2 enzymatic A peptides/chains and 2 non-enzymatic B peptides/chains. CFXIIIa is a dimer of activated A peptides/chains.

At least in dogs with kidney diseases CFXIII shows a deviation of the concentration, which is higher than or equal to (≥) 2 times the median of the standard concentration range, determined by mass spectrometry.

**Fibronectin (FN)** refers to a protein with protein ID J9P8M2. Fibronectin (FN) is a multifunctional adhesive glycoprotein that plays an important role in tissue repair, in regulating cell attachment and motility, and in embryogenesis.

At least in dogs with kidney diseases FN shows a deviation of the concentration, which is higher than or equal to (≥) 2 times the median of the standard concentration range, determined by mass spectrometry.

**Ficolin 1 (FIC)** refers to a protein with protein ID J9P7F7. Ficolin-1, also commonly termed M-ficolin, is a protein that in humans is encoded by the *FCN1* gene. Proteins of the ficolin family (ficolins) selectively recognize acetylated compounds. M-ficolin is predominantly expressed in the peripheral blood leukocytes and has been postulated to function as a plasma protein with elastin-binding activity.

At least in dogs with kidney diseases FIC shows a deviation of the concentration, which is higher than or equal to (≥) 2 times the median of the standard concentration range, determined by mass spectrometry.

**Hemopexin (HPX)** refers to a protein with protein ID F1PZR4. Hemopexin (or haemopexin; Hpx; Hx), also known as beta-1B-glycoprotein, is a glycoprotein that in humans is encoded by the *HPX* gene. Hemopexin is the plasma protein with the highest binding affinity for heme and some enzymatic activities.

At least in dogs with kidney diseases HPX shows a deviation of the concentration, which is higher than or equal to (≥) 2 times the median of the standard concentration range, determined by mass spectrometry.

**Inter-alpha-trypsin inhibitor heavy chain 4 (IATIH4)** refers to a protein with protein ID H9GWY3 (as listed in Table 1). Inter-alpha-trypsin inhibitors (IATI) are four plasma proteins consisting of four different heavy chains selected from the group IATIH1, IATIH2, IATIH3, IATIH4 and one light chain selected from the group AMBP or SPINT2 (also listed in Table 1). They function as protease inhibitors. The biomarker is **inter-alpha-trypsin inhibitor 4 (IATI4),** preferably **IATI heavy chain 4 (IATIH4).**

At least in dogs with kidney diseases IATI4 shows a deviation of the concentration, which is higher than or equal to (≥) 2 times the median of the standard concentration range, determined by mass spectrometry.

**Retinol-Binding Protein 4 (RBP4)** refers to a protein with protein ID F1Q4D9. Retinol binding protein 4, also known as RBP4, is a transporter protein for retinol. RBP4 is encoded by the *RBP4* gene in humans. It is mainly, synthesized in the liver and circulates in the bloodstream bound to retinol in a complex with transthyretin.

At least in dogs with kidney diseases RBP4 shows a deviation of the concentration, which is higher than or equal to (≥) 2 times the median of the standard concentration range, determined by mass spectrometry.

**Serum Amyloid A (SAA)** refers to a protein with protein ID J9NVE9. Serum amyloid A (SAA) proteins are a family of apolipoproteins associated with high-density lipoprotein (HDL) in plasma. Different isoforms of SAA are expressed constitutively (constitutive SAAs) at different levels or in response to inflammatory stimuli (acute phase SAAs). These proteins are produced predominantly by the liver.

At least in dogs with kidney diseases SAA shows a deviation of the concentration, which is higher than or equal to (≥) 2 times the median of the standard concentration range, determined by mass spectrometry.

**Talin 1 (Tal)** refers to a protein with protein ID J9P5V6. Talin-1 is a protein that in humans is encoded by the *TLN1* gene. Talin-1 is ubiquitously expressed and is localized to costamere structures in cardiac and skeletal muscle cells, and to focal adhesions in smooth muscle and non-muscle cells. Talin-1 functions to mediate cell-cell adhesion via the linkage of integrins to the actin cytoskeleton and in the activation of integrins.

At least in dogs with kidney diseases Tal shows a deviation of the concentration, which is higher than or equal to (≥) 2 times the median of the standard concentration range, determined by mass spectrometry.

**Kallikrein (KAL)** or Kallikrein B1 refers to a protein with protein ID F1PNV5. KLKB1 is a gene that, in humans, encodes the plasma kallikrein protein. Kallikreins are a subgroup of serine proteases.

At least in dogs with kidney diseases KAL shows a deviation of the concentration, which is higher than or equal to (≥) 2 times the median of the standard concentration range, determined by mass spectrometry.

**Dynein (DYN)** or dynein heavy chain domain 1, as listed in Table 1, refers to a protein with protein ID F1PZK8.

Dyneins are a family of cytoskeletal motor proteins that move along microtubules in cells. They convert the chemical energy stored in ATP to mechanical work. Dynein transports various cellular cargos, provides forces and displacements important in mitosis. Dyneins can be divided into two groups: cytoplasmic and axonemal dyneins. Dynein heavy chain domain 1 (DNAH1) belongs to the latter. The biomarker is dynein, preferably dynein heavy chain domain 1.

At least in dogs with kidney diseases DYN shows a deviation of the concentration, which is higher than or equal to (≥) 2 times the median of the standard concentration range, determined by mass spectrometry.

**Fukutin (FUK)** refers to a protein with protein ID E2R926. Fukutin is a eukaryotic protein necessary e.g. for the maintenance of muscle integrity, cortical histogenesis, and normal ocular development. Mutations in the fukutin gene have been shown to result in Fukuyama congenital muscular dystrophy (FCMD) characterised by brain malformation - one of the most common autosomal-recessive disorders in Japan. In humans this protein is encoded by the *FCMD* gene (also named *FKTN*).

At least in dogs with kidney diseases FUK shows a deviation of the concentration, which is higher than or equal to (≥) 2 times the median of the standard concentration range, determined by mass spectrometry.

**Myosin IXA (MYIXA)** refers to a protein with protein ID J9P187. Myosin IXa (Myo9a, previously also called myr7) is an actin-dependent motor protein of the unconventional myosin IX class.

At least in dogs with kidney diseases MYIXA shows a deviation of the concentration, which is higher than or equal to (≥) 2 times the median of the standard concentration range, determined by mass spectrometry.

**Kininogen 1** refers to a protein with protein ID E2R886. Kininogen-1 (KNG1), also known as alpha-2-thiol proteinase inhibitor, Williams-Fitzgerald-Flaujeac factor or the HMWK-kallikrein factor is a protein that in humans is encoded by the *KNG1* gene. Kininogen-1 is the precursor protein to high-molecular-weight kininogen (HMWK), low-molecular-weight kininogen (LMWK), and bradykinin.

At least in dogs with kidney diseases Kininogen 1 shows a deviation of the concentration, which is higher than or equal to (≥) 2 times the median of the standard concentration range, determined by mass spectrometry.

**α2-HS Glycoprotein** refers to a protein with protein ID E2QUV3. Alpha-2-HS-glycoprotein (AHSG, Alpha-2-Heremans-Schmid Glycoprotein) also known as fetuin-A is a protein that in humans is encoded by the *AHSG* gene. Fetuin-A belongs to the fetuin class of plasma binding proteins and is more abundant in fetal than adult blood.

At least in dogs with kidney diseases α2-HS glycoprotein shows a deviation of the concentration, which is higher than or equal to (≥) 2 times the median of the standard concentration range, determined by mass spectrometry.

**Complement C6** or complement component 6 refers to a protein with protein ID E2RGT6. C6 is one member of the complement membrane attack complex (MAC), which also includes complement components C5b, C7, C8 and C9.

At least in dogs with kidney diseases C6 shows a deviation of the concentration, which is higher than or equal to (≥) 2 times the median of the standard concentration range, determined by mass spectrometry.

**Complement C7** or complement component 7 refers to a protein with protein ID E2RG01. At least in dogs with kidney diseases C7 shows a deviation of the concentration, which is higher than or equal to (≥) 2 times the median of the standard concentration range, determined by mass spectrometry.

**Complement C8** or complement component 8 contains a protein with protein ID E2R109 (for the alpha chain), E2R141 (for the beta chain) and F6XKC0 (for the gamma chain) (as listed in Table 1). Complement component 8 is a protein involved in the complement system. It is part of the membrane attack complex (MAC). C8 is a heterotrimer; it consists of three different subunits. These are called C8 alpha, beta and gamma chains, encoded by the genes *C8A, C8B* and *C8G* respectively. The biomarker is Complement C8, preferably C8 alpha chain and/or C8 beta chain.

At least in dogs with kidney diseases C8 shows a deviation of the concentration, which is higher than or equal to (≥) 2 times the median of the standard concentration range, determined by mass spectrometry.

**Beta-2-glycoprotein 1 (APOH)** refers to a protein with protein ID P33703. $\beta_2$-glycoprotein 1, also known as beta-2 glycoprotein 1 and Apolipoprotein H (Apo-H), is a multifunctional plasma protein that in humans is encoded by the *APOH* gene. One of its functions is to bind cardiolipin. $\beta_2$-GP1 further has a complex involvement in agglutination.

At least in dogs with kidney diseases APOH shows a deviation of the concentration, which is higher than or equal to (≥) 2 times the median of the standard concentration range, determined by mass spectrometry.

**Serpin family A member 1** refers to a protein with protein ID F1PCE5. It is a serine protease inhibitor belonging to the serpin superfamily whose targets include elastase, plasmin, thrombin, trypsin, chymotrypsin, and plasminogen activator. This protein is produced in the liver, the bone marrow, by lymphocytic and monocytic cells in lymphoid tissue, and by the Paneth cells of the gut.

At least in dogs with kidney diseases serpin family A member 1 shows a deviation of the concentration, which is higher than or equal to (≥) 2 times the median of the standard concentration range, determined by mass spectrometry.

**Transferrin** refers to a protein with protein IDs J9P430 and F6V1W9. Transferrins are glycoproteins found in vertebrates which contain binding sites for two $Fe^{3+}$ ions, bind to and consequently mediate the transport of iron (Fe) through blood plasma (serotransferrin). Additionally, transferrins and fragments therefrom possess many immune regulatory activities. They are produced predominantly in the liver, but also in many other organs. Human transferrin is encoded by the *TF* gene.

At least in dogs with kidney diseases transferrin shows a deviation of the concentration, which is higher than or equal to (≥) 2 times the median of the standard concentration range, determined by mass spectrometry.

α1-**B Glycoprotein** refers to a protein with protein ID F1PCK2. Alpha-1-B glycoprotein is a protein in humans that is encoded by the A1BG gene. The protein encoded by this gene is a plasma glycoprotein of unknown function. The protein shows sequence similarity to the variable regions of some immunoglobulin supergene family member proteins.

At least in dogs with kidney diseases α1-B glycoprotein shows a deviation of the concentration, which is higher than or equal to (≥) 2 times the median of the standard concentration range, determined by mass spectrometry.

**Apo AI** or apolipoprotein AI refers to a protein with protein ID J9P843. Apolipoprotein AI (Apo-AI) is a protein that in humans is encoded by the *APOA1* gene. As the major component of HDL particles, it has a specific role in lipid metabolism.

At least in dogs with kidney diseases Apo AI shows a deviation of the concentration, which is higher than or equal to (≥) 2 times the median of the standard concentration range, determined by mass spectrometry.

**Apo D** or apolipoprotein D refers to a protein with protein ID E2RNM1. Apolipoprotein D (ApoD) is a protein that in humans is encoded by the *APOD* gene. ApoD is a member of the lipocalin family, small hydrophobic molecule transporters.

At least in dogs with kidney diseases Apo D shows a deviation of the concentration, which is higher than or equal to (≥) 2 times the median of the standard concentration range, determined by mass spectrometry.

**Alpha-1-microglobulin/bikunin precursor** refers to a protein with protein ID J9NUI6. Protein AMBP is a protein that binds to IATIs and in humans is encoded by the *AMBP* gene.

At least in dogs with kidney diseases alpha-1-microglobulin/bikunin precursor shows a deviation of the concentration, which is higher than or equal to (≥) 2 times the median of the standard concentration range, determined by mass spectrometry.

**Carboxypeptidase N subunit 2** refers to a protein with protein ID J9PAA4. Carboxypeptidase N subunit 2 is an enzyme that in humans is encoded by the *CPN2* gene.

At least in dogs with kidney diseases carboxypeptidase N subunit 2 shows a deviation of the concentration, which is higher than or equal to (≥) 2 times the median of the standard concentration range, determined by mass spectrometry.

**Serine/arginine repetitive matrix protein 3** refers to a protein with protein ID F6Y120. Serine/arginine repetitive matrix protein 1 is a protein that in humans is encoded by the *SRRM1* gene. Serine/arginine repetitive matrix protein 3 is listed as Serine/arginine repetitive matrix protein 3 (homolog) in Table 1.

At least in dogs with kidney diseases serine/arginine repetitive matrix protein 1 shows a deviation of the concentration, which is higher than or equal to (≥) 2 times the median of the standard concentration range, determined by mass spectrometry.

Before mass spectrometry all proteins have been denatured and digested by trypsin (as described in Section 1.4. LC-MS/MS analysis of Example 1 below) into characteristic tryptic peptides. Thus, after analysis of all these peptides and database search the mass spectrometric identified biomarker candidates appear as protein chain(s), multiple variants, and/or fragments (see Table 1 and also Section 1.6 of Example 1 below). All biomarkers of the present invention derived from these findings are designated as proteins appearing in non-denatured real clinical samples suitable for diagnostic purposes. As discussed above, the term "protein", as used herein, refers to / comprises all known proteins enclosing the identified chains of the respective protein, modified variants of the respective protein, fragments of the respective protein, single protein chains, multiple protein chains and complexes of the respective protein (chain) with further proteins and protein chains, irrespective of their validation using a currently available immune assay like ELISA.

In one embodiment, the at least one further protein is preferably selected from:

Fibrinogen, preferably fibrinogen γ-chain (FGG),
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP), and
C Reactive Protein (CRP),
α-1-Acid Glycoprotein (a1AGP),
Coagulationfactor XIII (CFXIII),
Complement Component 1q (C1q),

Complement Component 9 (C9),
Fibronectin (FN),
Ficolin 1 (FIC),
Hemopexin (HPX),
Inter-alpha-trypsin inhibitor 4 (IATI4), preferably IATI heavy chain 4,
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN), preferably dynein heavy chain domain 1,
Fukutin (FUK), and
Myosin IXA (MYIXA),

the at least one further protein is more preferably selected from:

Fibrinogen, preferably fibrinogen γ-chain (FGG),
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP), and
C Reactive Protein (CRP).

In a preferred embodiment

C4BP and ADP, or
COLXIII and FMN, or
HABP2 and TGF-β1, or
HABP2 and FGG, or
CFH and GS, or
CFI and GS, or
CFI and VTN, or
CFH and FGG, or
CFI and FGG

are determined.

Furthermore, the present invention provides *at least two proteins* selected from

Collagen type XIII (COLXIII),
Hyaluronan-Binding Protein 2 (HABP2),
C4 Binding Protein (C4BP), preferably C4BP alpha chain (C4BPA),
Complement Factor H (CFH),
Complement Factor I (CFI),
Fibrinogen, preferably fibrinogen γ-chain (FGG),
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP),
C Reactive Protein (CRP),
α-1-Acid Glycoprotein (a1AGP),

Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4), preferably IATI heavy chain 4 (IATIH4),
Leucine Rich $\alpha$-2-Glycoprotein 1 (LRGP1),
Retinol-Binding Protein 4 (RBP4),
Serum Amyloid A (SAA),
Talin 1 (Tal),
Kallikrein (KAL),
Dynein (DYN), preferably dynein heavy chain domain 1,
Fukutin (FUK),
Myosin IXA (MYIXA),
Kininogen 1,
$\alpha$2-HS Glycoprotein,
Complement C6,
Complement C7,
Complement C8, preferably C8 alpha chain and/or C8 beta chain,
APOH, Beta-2-glycoprotein 1,
Serpin family A member 1,
Transferrin,
$\alpha$1-B Glycoprotein,
Apo AI,
Apo D,
Alpha-1-microglobulin/bikunin precursor,
Carboxypeptidase N subunit 2,
Serine/arginine repetitive matrix protein 3,
Complement C1s,
Complement C1r,
Complement C2,
Complement C3,
Complement C4,
Complement C5,
Complement factor B,
Haptoglobin,
$\alpha$2-macroglobulin,
Alpha-1-antitrypsin,
Ceruloplasmin,
Serpin family A member 3,
Serpin family A member 5,
Serpin family A member 7,
Serpin family G member 1,
Serpin family D member 1,
Coagulation factor V,
Prothrombin,
Plasminogen,
Protein S,
Afamin,
Transthyretin,
Hemoglobin subunit alpha,
Fetuin B,
Insulin like growth factor binding protein,
Alpha-2-glycoprotein 1,
Paraoxonase 1,
Dipeptidyl peptidase 4,
C-type lectin domain family 3 member B,

Galectin 3 binding protein,
Apolipoprotein AIV (Apo AIV),
Apolipoprotein CI (Apo CI),
Apolipoprotein CII (Apo CII),
Apolipoprotein E (Apo E4),
Apolipoprotein M (Apo M),
Apolipoprotein B (Apo B),
Myosin IB,
Myosin XVIIIB,
Kinesin-like protein,
CD109 molecule,
Maltase-glucoamylase,
Sacsin molecular chaperone,
Dispatched RND transporter family member 2,
DNA Polymerase,
Trafficking protein particle complex subunit 2,
RB associated KRAB zinc finger,
Ciliogenesis-associated TTC17-interacting protein,
Proteasome subunit alpha type,
Regulating synaptic membrane exocytosis 2,
Extracellular matrix protein 1,
Cadherin 5,
Coiled-coil domain containing 178, and
Attractin,

for use as biomarker in a method for the pre-clinical and/or early-stage detection and/or diagnosis of a kidney disease.

Said *at least two proteins* are preferably selected from

Collagen type XIII (COLXIII),
Hyaluronan-Binding Protein 2 (HABP2),
C4 Binding Protein (C4BP), preferably C4BP alpha chain (C4BPA),
Complement Factor H (CFH),
Complement Factor I (CFI),
Fibrinogen, preferably fibrinogen γ-chain (FGG),
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP),
C Reactive Protein (CRP),
α-1-Acid Glycoprotein (a1AGP),
Coagulationfactor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4), preferably IATI heavy chain 4 (IATIH4),
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN), preferably dynein heavy chain domain 1,
Fukutin (FUK), and

Myosin IXA (MYIXA).

In a preferred embodiment

ADP and PICP, or
AGT and GS, or
AGT and C9, or
GS and C9, or
GS and VTN, or
AGT and VTN, or
CFI and a1AGP, or
LRGP1 and C1q, or
CFH and C9, or
CRP and C1q

are determined.

Preferably, the concentration of each of the proteins is determined in a sample of a mammal, preferably a human (subject).

The mammal is preferably

a human,
an animal, such as a dog, cat, rabbit, guinea pig, hamster, cattle, pig, horse, sheep, donkey, goat, red deer, or camel,

more preferably the mammal is a human.

The sample is preferably a bodily fluid, such as urine or blood, e.g. blood serum, blood plasma or whole blood.

The bodily fluid is more preferably urine.

In one embodiment, where the sample is a urine sample, also the creatine concentration of said urine sample is determined and then the concentration of the biomarker protein is normalized against said creatine concentration.

Preferably, the normalized concentration of each of the proteins is compared to a control value.

Preferably, the control value is determined for each protein from healthy subjects, preferably a group of healthy subjects matching the subject under diagnostics regarding age and gender. A "subject" or preferably a "human subject" is considered a "healthy subject" if he/she is "kidney-healthy" at the time a sample is taken and also stays "kidney-healthy" for a certain time that corresponds to the usual time during which a kidney disease will become diagnosable by usual means. Biobanks, for example, are suitable for obtaining samples of healthy subjects, since their subjects are followed up after samples are taken.

Preferably, the control value is the median of a standard concentration range.

In a preferred embodiment, a mathematical link of the concentrations of the biomarker is used, such as the sum of the concentrations or, preferably, the product of the concentrations.

For example, the following products are built ADP x C4BP, ColXIII x C4BP, ColXIII x FMN, HABP2 x TGF-$\beta$1, HABP2 x FGG, ColXIII x C4BP x HABP2.

*Detection methods*

As discussed above, the present invention provides a method for the pre-clinical and/or early-stage detection and/or diagnosis of a kidney disease, comprising

(a) determining the concentrations of at least two proteins in a sample of a mammal,
(b) comparing each of the concentrations determined in step (a) with a control value, wherein a deviation of each of the concentrations determined in step (a) from said control value is indicative of a pre-clinical and/or early-stage kidney disease.

Preferably, the kidney disease is selected from chronic progressive kidney diseases

starting with glomerular injury (glomerulopathy),
preferably post-infectious glomerulopathy, IgA-nephropathy, Henoch-Schoenlein-Purpura, Focal segmental glomerulosclerosis, Alport syndrome, thin basement membrane nephropathy, benign familiar hematuria,
or
starting on other parts of the body, with other dysregulations, and/or other parts of the kidney with implication on the glomerulus (nephropathy),

preferably metabolic syndrome, hypertension, heart failure, nephrotoxic medication, adipositas, diabetes mellitus (diabetic nephropathy), renal agenesis, renal hypoplasia, multicystic renal dysplasia, autosomal dominant polycystic disease, autosomal recessive polycystic disease, anomalies of the urinary tract, duplex kidneys, nephronophthisis.

- Step (a)

In step (a) the concentrations of at least two proteins are determined in a sample of a mammal.

The mammal is preferably

a human,
an animal, such as a dog, cat, rabbit, guinea pig, hamster, cattle, pig, horse, sheep, donkey, goat, red deer, or camel,

more preferably the mammal is a human.

The sample is preferably a bodily fluid, such as urine or blood, e.g. blood serum, blood plasma or whole blood.

The bodily fluid is more preferably urine

In one embodiment, where the sample is a urine sample, also the creatine concentration of said urine sample is determined and then the concentration of the biomarker protein is normalized against said creatine concentration.

The at least two proteins are selected from the biomarkers disclosed herein.

In a preferred embodiment, said at least two proteins are selected from

Collagen type XIII (COLXIII),
Hyaluronan-Binding Protein 2 (HABP2),
C4 Binding Protein (C4BP), preferably C4BP alpha chain (C4BPA),
Complement Factor H (CFH), and
Complement Factor I (CFI).

In a preferred embodiment, said at least two proteins are selected from

- one protein which selected from

Collagen type XIII (COLXIII),
Hyaluronan-Binding Protein 2 (HABP2),
C4 Binding Protein Alpha (C4BP), preferably C4BP alpha chain (C4BPA),
Complement Factor H (CFH), and
Complement Factor I (CFI),

and at least one further protein which is selected from:

- Fibrinogen, preferably fibrinogen γ-chain (FGG),
- Lumican (LUM),
- Formin 1 (FMN),
- Vitronectin (VTN),
- Gelsolin (GS),
- Angiotensinogen (AGT),
- Adiponectin (ADP),
- Transforming growth factor-β1 (TGF-β1),
- Carboxyterminal Propeptid of Type I Procollagen (PICP),
- C Reactive Protein (CRP),
- α-1-Acid Glycoprotein (a1AGP),
- Coagulation factor XIII (CFXIII),
- Complement Component 1q (C1q),
- Complement Component 9 (C9),
- Fibronectin (FN),
- Ficolin 1 (FIC),
- Hemopexin (HPX),
- Inter-alpha-trypsin inhibitor 4 (IATI4), preferably IATI heavy chain 4,
- Leucine Rich α-2-Glycoprotein 1 (LRGP1),

- Retinol-Binding Protein 4 (RBP4),
- Serum Amyloid A (SAA),
- Talin 1 (Tal),
- Kallikrein (KAL),
- Dynein (DYN), preferably dynein heavy chain domain 1,
- Fukutin (FUK),
- Myosin IXA (MYIXA).
- Kininogen 1,
- α2-HS Glycoprotein,
- Complement C6,
- Complement C7,
- Complement C8, preferably C8 alpha chain and/or C8 beta chain,
- APOH, Beta-2-glycoprotein 1,
- Serpin family A member 1,
- Transferrin,
- α1-B Glycoprotein,
- Apo AI,
- Apo D,
- Alpha-1-microglobulin/bikunin precursor,
- Carboxypeptidase N subunit 2, and
- Serine/arginine repetitive matrix protein 3.

In one embodiment, the at least one further protein is preferably selected from:

- Fibrinogen, preferably fibrinogen γ-chain (FGG),
- Lumican (LUM),
- Formin 1 (FMN),
- Vitronectin (VTN),
- Gelsolin (GS),
- Angiotensinogen (AGT),
- Adiponectin (ADP),
- Transforming growth factor-β1 (TGF-β1),
- Carboxyterminal Propeptid of Type I Procollagen (PICP),
- C Reactive Protein (CRP),
- α-1-Acid Glycoprotein (a1AGP),
- Coagulation factor XIII (CFXIII),
- Complement Component 1q (C1q),
- Complement Component 9 (C9),
- Fibronectin (FN),
- Ficolin 1 (FIC),
- Hemopexin (HPX),
- Inter-alpha-trypsin inhibitor 4 (IATI4), preferably IATI heavy chain 4,
- Leucine Rich α-2-Glycoprotein 1 (LRGP1),
- Retinol-Binding Protein 4 (RBP4),
- Serum Amyloid A (SAA),
- Talin 1 (Tal),
- Kallikrein (KAL),
- Dynein (DYN), preferably dynein heavy chain domain 1,
- Fukutin (FUK), and
- Myosin IXA (MYIXA).

In a preferred embodiment, the at least one further protein is selected from:

Fibrinogen, preferably fibrinogen γ-chain (FGG),
Lumican (LUM),
Formin 1 (FMN),
Vitronectin (VTN),
Gelsolin (GS),

Angiotensinogen (AGT),
Adiponectin (ADP),
Transforming growth factor-$\beta$1 (TGF-$\beta$1),
Carboxyterminal Propeptid of Type I Procollagen (PICP), and
C Reactive Protein (CRP).

In a preferred embodiment, said at least two proteins are selected from

Collagen type XIII (COLXIII),
Hyaluronan-Binding Protein 2 (HABP2),
C4 Binding Protein (C4BP), preferably C4BP alpha chain (C4BPA),
Complement Factor H (CFH),
Complement Factor I (CFI),
Fibrinogen, preferably fibrinogen $\gamma$-chain (FGG),
Lumican (LUM),
Formin 1 (FMN),
Vitronectin (VTN),
Gelsolin (GS),
Angiotensinogen (AGT),
Adiponectin (ADP),
Transforming growth factor-$\beta$1 (TGF-$\beta$1),
Carboxyterminal Propeptid of Type I Procollagen (PICP),
C Reactive Protein (CRP),
$\alpha$-1-Acid Glycoprotein (a1AGP),
Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4), preferably IATI heavy chain 4,
Leucine Rich $\alpha$-2-Glycoprotein 1 (LRGP1),
Retinol-Binding Protein 4 (RBP4),
Serum Amyloid A (SAA),
Talin 1 (Tal),
Kallikrein (KAL),
Dynein (DYN), preferably dynein heavy chain domain 1,
Fukutin (FUK),
Myosin IXA (MYIXA),
Kininogen 1,
$\alpha$2-HS Glycoprotein,
Complement C6,
Complement C7,
Complement C8, preferably C8 alpha chain and/or C8 beta chain,
APOH, Beta-2-glycoprotein 1,
Serpin family A member 1,
Transferrin,
$\alpha$1-B Glycoprotein,
Apo AI,
Apo D,
Alpha-1-microglobulin/bikunin precursor,
Carboxypeptidase N subunit 2,
Serine/arginine repetitive matrix protein 3,
Complement C1s,
Complement C1r,
Complement C2,
Complement C3,
Complement C4,

Complement C5,
Complement factor B,
Haptoglobin,
$\alpha$2-macroglobulin,
Alpha-1-antitrypsin,
Ceruloplasmin,
Serpin family A member 3,
Serpin family A member 5,
Serpin family A member 7,
Serpin family G member 1,
Serpin family D member 1,
Coagulation factor V,
Prothrombin,
Plasminogen,
Protein S,
Afamin,
Transthyretin,
Hemoglobin subunit alpha,
Fetuin B,
Insulin like growth factor binding protein,
Alpha-2-glycoprotein 1,
Paraoxonase 1,
Dipeptidyl peptidase 4,
C-type lectin domain family 3 member B,
Galectin 3 binding protein,
Apolipoprotein AIV (Apo AIV),
Apolipoprotein CI (Apo CI),
Apolipoprotein CII (Apo CII),
Apolipoprotein E (Apo E4),
Apolipoprotein M (Apo M),
Apolipoprotein B (Apo B),
Myosin IB,
Myosin XVIIIB,
Kinesin-like protein,
CD109 molecule,
Maltase-glucoamylase,
Sacsin molecular chaperone,
Dispatched RND transporter family member 2,
DNA Polymerase,
Trafficking protein particle complex subunit 2,
RB associated KRAB zinc finger,
Ciliogenesis-associated TTC17-interacting protein,
Proteasome subunit alpha type,
Regulating synaptic membrane exocytosis 2,
Extracellular matrix protein 1,
Cadherin 5,
Coiled-coil domain containing 178, and
Attractin.

In one embodiment, said at least two proteins are preferably selected from:

Collagen type XIII (COLXIII),
Hyaluronan-Binding Protein 2 (HABP2),
C4 Binding Protein (C4BP), preferably C4BP alpha chain (C4BPA),
Complement Factor H (CFH),
Complement Factor I (CFI),
Fibrinogen, preferably fibrinogen $\gamma$-chain (FGG),
Lumican (LUM),

Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP),
C Reactive Protein (CRP),
α-1-Acid Glycoprotein (a1AGP),
Coagulation factor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4), preferably IATI heavy chain 4,
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN), preferably dynein heavy chain domain 1,
Fukutin (FUK), and
Myosin IXA (MYIXA).

In one embodiment, the concentrations of

COLXIII and C4BP, or
COLXIII and C4BP and HABP2, or
CFH and CFI are determined.

In one embodiment, the concentrations of COLXIII, HABP2, C4BP, CFH, and CFI are determined.
In one embodiment, the concentrations of

C4BP and ADP, or
COLXIII and FMN, or
HABP2 and TGF-β1, or
HABP2 and FGG, or
CFH and GS, or
CFI and GS, or
CFI and VTN, or
CFH and FGG, or
CFI and FGG are determined.

In one embodiment, the concentrations of

ADP and PICP, or
AGT and GS, or
AGT and C9, or
GS and C9, or
GS and VTN, or
AGT and VTN, or
CFI and a1AGP, or
LRGP1 and C1q, or
CFH and C9, or
CRP and C1q are determined.

*- Step (b)*

In step (b), each of the concentrations determined in step (a) are compared with a control value.

In one embodiment, where the sample is a urine sample, also the creatine concentration of said urine sample is determined and then the concentration of the biomarker protein is normalized against said creatine concentration.

Preferably, the normalized concentration of each of the proteins is compared to a control value.

Preferably, the control value is determined for each protein from healthy subjects, preferably a group of healthy subjects matching the subject under diagnostics regarding age and gender. As discussed above, a subject is considered a "healthy subject" if he/she is "kidney-healthy" at the time a sample is taken and also stays "kidney-healthy" for a certain time that corresponds to the usual time during which a kidney disease will become diagnosable by usual means. Biobanks, for example, are suitable for obtaining samples of healthy subjects, since their subjects are followed up after samples are taken.

Preferably, the control value is the median of a standard concentration range.

According to the invention, a deviation of each of the concentrations determined in step (a) from said control value is indicative of a pre-clinical and/or early-stage kidney disease.

Preferably,

a deviation higher than or equal to ($\geq$) 2 times the median of the standard concentration range is indicative of a pre-clinical and/or early-stage kidney disease; or

a deviation lower than or equal to ($\leq$) 0.5 times the median of the standard concentration range is indicative of a pre-clinical and/or early-stage kidney disease.

More preferably,

a deviation higher than or equal to ($\geq$) 2 times the median of the standard concentration range is indicative of a pre-clinical and/or early-stage kidney disease.

A deviation higher than or equal to ($\geq$) 2 times the median of the standard concentration range can be in the range from 2-times to about 1.000-times, such as 2-times to 100-times, such as 2-times to 50-times or 2-times to 10-times.

In a preferred embodiment, a mathematical link of the concentrations of the biomarker is used, such as the sum of the concentrations or, preferably, the product of the concentrations.

For example, the following products are built ADP x C4BP, ColXIII x C4BP, ColXIII x FMN, HABP2 x TGF-$\beta$1, HABP2 x FGG, ColXIII x C4BP x HABP2.

*Further description of preferred embodiments*

Although several early cellular alterations have been uncovered recently (see e.g., Guo *et al.,* 2019), up to date there is no practicable biomarker in use for the pre-clinical detection of Alport syndrome (AS).

With manifested AS we identified a panel of increased biomarker (BM) candidates using our proteomic workflow and ELISA (Baum *et al.,* 2008; Pohl *et al.,* 2013). Unfortunately, a similar search for BMs in a pre-clinical state, i.e. in toddlers, is not possible for ethical and practical reasons.

However, type IV collagens are highly conserved. Hence, any mammals can suffer from AS, making AS animal models ideal tools to study pathogenesis and therapy (Kashtan *et al.,* 2002).

The mouse and the canine models show a similar clinical course compared to humans. In addition, ACE-inhibitors were shown to delay disease progression in both COL4A5[-] dogs (Grodecki *et al.,* 1997) (AS-dogs) and in COL4A3[-/-] mice (Gross *et al.,* 2003) (AS-mice) prior to extensive assessment in humans. In these models definitive diagnosis in the preclinical stage of disease is allowed by genetic means. Biomarkers in body fluids indicating disease progression also could be observed in these models. Thus, our improved approach (Wendler *et al.,* 2013) has been already applied to serum of juvenile AS-mice resulting in some candidate BM (Muckova *et al.,* 2015).

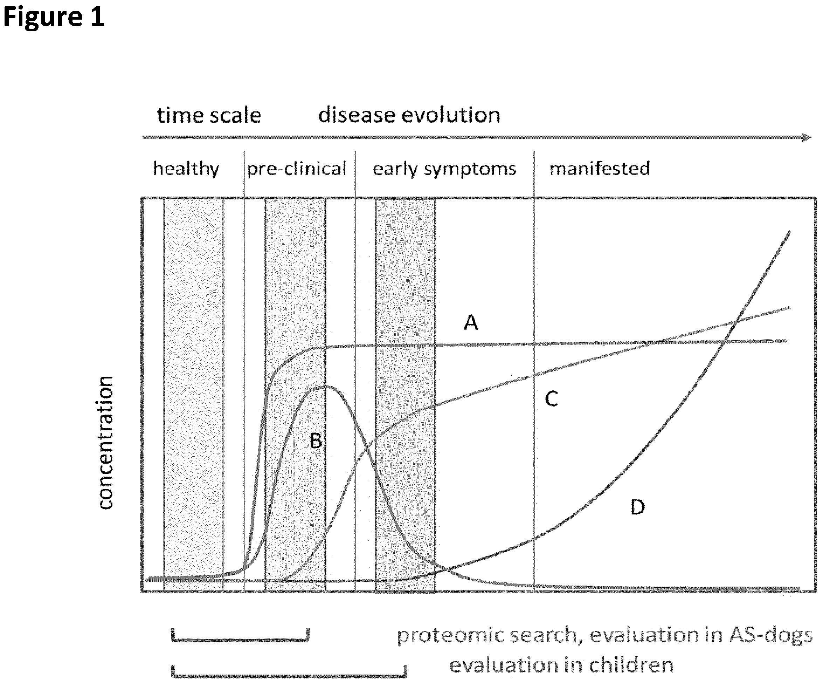

So far, most described biomarker candidates resulted from hypothesis-driven studies. For pre-clinical processes a hypothesis hardly can be given. Hence, a hypothesis-free search for reliable biomarkers is an opportunity to identify novel biomarkers for diagnosis, prognosis, and therapeutic monitoring of nephropathies, which are urgently required. The search for pre-clinical biomarkers is highly challenging. These biomarkers only could be searched for and evaluated in seemingly healthy but affected pre-clinical individuals exhibiting traits of a disease that predictably progress to nephropathy (see Figure 1).

AS, serving as a model genetic disease, was subjected to proteomic search for such early biomarkers, see below. This search was performed in juvenile AS-mice and AS-dogs. Most of the identified biomarker candidates can be considered "verified", since they appear overlapping in two different species (see Table 1). Moreover, some show reversibility under therapy with an ACE-inhibitor (Ramipril) and some of them have been evaluated by immune assays in mice and dogs. We further verified a panel of these biomarker candidates in DM1 with expectable pre-clinical DN and children with obesity, hypertension and metabolic syndrome (potentially pre-clinical DM) and other nephropathies in their early stage.

The present specification discloses a methodologically complementary proteomic study, using easily accessible blood serum for biomarker search in a clearly pre-clinical stage of the model disease Alport syndrome (AS) and a verification in blood and urine specimens from pediatric patients with highly probable pre-clinical conditions for the development of a DN, and with very early clinical stages of various nephropathies.

To this end, an optimized and unbiased strategy was applied combining

i. sophisticated sample collection from an animal model (dogs) of an inherited glomerulopathy, the Alport syndrome (AS), for pre-clinical proteomic search, and from children without and with already diagnosed early nephropathies, for verification,

ii. application of a comprehensive and precise workflow to proteomic biomarker search using dog samples from affected male and female dogs of 7 weeks as well as their non-affected siblings, and

iii. subsequent evaluation of biomarkers using a human biobank implicating specimens of 305 characterized children including healthy controls, children with metabolic syndrome, hypertension, adipositas, T2DM, T1DM, renal agenesis, hypoplasia. multicystic renal dysplasia, anomalies of the urinary tract, duplex kidneys, ADPKD, ARPKD, nephronophthisis, post-infectious GN, vasculitis (IgAN, HSP), thin basement membrane nephropathy and benign familar hematuria, and AS.

*Table 1*    Mass spectrometric indentified biomarker candidates in mice and dogs

| altered proteins in dogs (7. wk) | | males | | females | | BM candidats | | | | verified | | |
| | | mean ratios | | | | | | | | Ø: no, •: yes | | |
| | | higher in AM | | higher in CF | | mice wk | | dogs wk 7 | | mice | dogs | man |
| ID* | annotation | in sub-fractions | overall trend | in sub-fractions | overall trend | 4 | 6-7.5 | AM | CF | | | |
| Plasma proteins | | | | | | | | | | | | |
| F1PNV5 | Kallikrein B1 | DIV/0 | 114,84 | 0 | 0 | | x | x | | | Ø | |
| E2R886 | Kininogen 1 | 56,2 | 2,08 | 20,3 | 1,35 | x | x | x | o | | | |
| E2QUV3 | α2-HS glycoprotein | 326 | 16,25 | n.a. | | x | x | x | | | | |
| F1P6E1 | Complement C1s | 0 | 0 | 3,11 | DIV/0 | | | | x | | | |
| F1PNG7 | Complement C1r | n.a. | | 115 | 9,99 | | | | x | | | |
| J9P4B4 | Complement C1q A chain | DIV/0 | 35,13 | n.f. | | | | x | | | | |
| J9P1G2 | Complement C1q B chain | DIV/0 | 107,34 | 4,09 | 0,16 | | | x | | | | |
| E2RJC2 | Complement C1q C chain | DIV/0 | 78,88 | 3,21 | 0,07 | | | x | | | | • |
| E2RS79 | Complement C2 (human homolog) | 3,44 | 0,13 | 0 | 0 | | x | | | | | |
| F1PIX8 | Complement C3 | 32,2 | 0,46 | 34,77 | 1,54 | o | x | o | o | | | |
| J9PAD1 | Complement C4 (human homolog) | 29,6 | 0,22 | 150 | 0,93 | | x | o | o | | | |
| F1PWR2 | Complement C4 (human homolog) | 6,09 | 0,20 | 37,3 | 0,48 | | x | o | o | | | |
| F1P7J4 | Complement C5 | 21,3 | 0,12 | 14,9 | 0,13 | | | o | o | | | |
| E2RGT6 | Complement C6 | DIV/0 | 34,84 | 0 | 0 | | | x | | | | |
| E2RG01 | Complement C7 | DIV/0 | 34,21 | 0 | 0 | | | x | | | | |
| E2R109 | Complement C8 alpha chain | DIV/0 | 44,49 | 2,26 | 0 | | | x | | | | |
| E2R141 | Complement C8 beta chain | 2,41 | 0,02 | n.a. | | | | | | | | |
| J9P8Z6 | Complement C9 | 5,79 | DIV/0 | n.a. | | | x | x | | | | • |
| F1PY40 | Complement factor I | 66,2 | 1,13 | 49,5 | 0,60 | | x | o | o | | | • |
| J9NTL7 | Complement factor H (homolog in mammals) | 69,5 | 0,47 | 47,6 | 0,08 | | x | o | o | | | • |
| E2RS80 | Complement factor B (homolog in primates) | 39 | 0,91 | 25 | 0,29 | | | o | o | | | |

EP 4 397 976 A1

EP 4 397 976 A1

| ID | Protein | | | | | | | | | | | | |
|---|---|---|---|---|---|---|---|---|---|---|---|---|---|
| F1PGM9 | Complement component 4 binding protein alpha | 170,4 | 13,87 | 20,2 | 0,47 | | | x | o | | | | ● |
| G1K2D9 | Haptoglobin | 114 | 4,88 | 0 | 0 | x | x | x | | | | | ● |
| F6UME0 | α2-macroglobulin | 77,55 | 2,48 | 179 | 1,08 | | x | x | o | | | | ● |
| F6Y713 | α1-acid glycoprotein | 23 | 9,31 | 13,6 | 0,07 | | | x | o | | | | ● |
| P33703 | APOH, Beta-2-glycoprotein 1 | 42,2 | 3,76 | 15,7 | 0,41 | x | x | x | o | | | | ● |
| T2KEN6 | Pentraxin (CRP) | 3,53 | 0,06 | 0 | 0 | x | | | | | | | ● |
| A1ILJ0 | Alpha-1-antitrypsin | 6,2 | 1,33 | 4,08 | 0,10 | x | x | o | | | | | |
| F1PAX2 | Ceruloplasmin | 4,38 | 0,07 | 6,28 | 0,07 | | x | o | o | | | | |
| F1PCE5 | Serpin family A member 1 | 114 | 5,04 | 53,1 | 2,93 | x | | x | x | | | | |
| F1PH86 | Serpin family A member 3 | n.f. | | 2,4 | 0,11 | | | | | | | | |
| E2RMF9 | Serpin family A member 5 | 0 | 0 | 34 | 1,33 | | | o | | | | | |
| F1PB85 | Serpin family A member 7 | 8,55 | 2,48 | 0 | 0 | | | x | | | | | |
| F1PYX9 | Serpin family G member 1 | 11,3 | 0,14 | 12,9 | 0,08 | | | o | o | | | | |
| J9P8Q8 | Serpin family D member 1 | 2,17 | 0,02 | 2,53 | 0,28 | | | | | | | | |
| E2R833 | Leucine rich alpha-2-glycoprotein 1 | 22 | 13,15 | 10,6 | 0,43 | x | x | x | o | | ● | ● | |
| J9NVE9 | Serum amyloid A | 86,4 | DIV/0 | 0 | 0 | x | x | x | | | ● | Ø | |
| F1PAL5 | Angiotensinogen | 19,4 | 3,59 | 6,4 | 0,83 | x | x | x | o | | | ● | |
| F1PN98 | Coagulation factor V | 3,35 | DIV/0 | 0 | 0 | | | | | | | | |
| J9NSF9 | Prothrombin | 51 | 3,18 | 11,9 | 0,25 | | | x | o | | | | |
| F1PZR4 | Hemopexin | 44,9 | 0,90 | 68,7 | 1,41 | | o | o | o | | | | Ø |
| F1Q421 | Plasminogen | n.a. | | 52,6 | 3,18 | x | x | | x | | | | |
| J9P430 | Transferrin | 20,4 | 0,56 | 29,1 | 0,17 | x | o | o | o | | | | |
| F6V1W9 | Transferrin | 4,12 | 3,04 | 3,95 | 0,91 | | | x | o | | | | |
| J9JHX7 | Serotransferrin (homolog, several species) | 9,23 | 1,45 | 20,2 | 0,90 | | | o | o | | | | |
| E2QZQ1 | Serotransferrin (homolog, several species) | 6,75 | 0,53 | DIV/0 | 40,50 | | | o | x | | | | |
| F1PKX3 | Coagulation factor XIII A chain | 2,76 | 0,02 | 41,2 | 44,06 | | | | x | | | | |
| F1Q041 | Coagulation factor XIII B chain | DIV/0 | 3,95 | DIV/0 | 21,47 | | | x | x | | | | Ø |
| F1PSS8 | Protein S | 2,98 | 0,06 | 0 | 0 | | | | | | | | |
| E2R4E7 | Afamin | 6,76 | 0,59 | 14 | 0,23 | | x | o | o | | | | |
| F6Y3P9 | Gelsolin | 34,5 | 3,68 | 20 | 3,67 | x | | x | x | ● | ● | ● | |

| Accession | Protein | | | | | | | | | |
|---|---|---|---|---|---|---|---|---|---|---|
| E2R5U8 | Transthyretin | 8,76 | 0,11 | n.a. | | | x | | o | |
| J9NXL3 | Hemoglobin subunit alpha (dog homolog) | 115 | 34,62 | 2,8 | 0,01 | | x | | x | |
| E2R9B6 | Fetuin B | 30,4 | 4,69 | 2 | 0,03 | | | | x | |
| F1PYM4 | Insulin like growth factor binding protein | 31 | 0,82 | 3,15 | 1,59 | | | | o | |
| B5U1S6 | Adiponectin (fragment) | DIV/0 | 51,86 | DIV/0 | 1,93 | | | | x | o | • • |
| F1PDJ7 | Alpha-2-glycoprotein 1 | 3,75 | 1,99 | n.a. | | | | | | |
| E2RPW3 | Paraoxonase 1 | 3,55 | 0,08 | DIV/0 | 264,38 | | | | | x |
| J9P8B0 | Dipeptidyl peptidase 4 | 2,31 | DIV/0 | n.a. | | | | | | |
| F1PCK2 | α1-B glycoprotein | 21,9 | 0,67 | 25,2 | 1,85 | | | | o | o |
| F1Q4D9 | Retinol binding protein 4 | 8,97 | 0,20 | 25,06 | 4,95 | x | | | o | x | Ø |
| E2RPB8 | C-type lectin domain family 3 member B | n.a. | | 2,59 | 0,06 | | | | | |
| E2RKQ6 | Galectin 3 binding protein | 2,24 | 1,70 | 3,3 | 0,08 | | | | | |
| **Apolipoproteins** | | | | | | | | | | |
| J9P843 | Apo AI | 325 | 12,75 | 41,1 | 0,60 | x | x | | x | o |
| E2RE76 | Apo AIV | 6,76 | 0,44 | 21,5 | 5,07 | x | x | | o | x |
| P56595 | Apo CI | 4,72 | 0,05 | 3,97 | DIV/0 | | | | | |
| 9NWJ6 | Apo CII | 15,8 | 0,18 | 50,3 | 12,99 | | | | o | x |
| D5G334 | Apo E4 (fragment) | 4,8 | 0,16 | 37,4 | 4,13 | | | | | o |
| F1PJ74 | Apo E | 15 | 0,34 | 33 | 2,93 | x | x | | o | x |
| E2RQ71 | Apo M | 6,86 | 0,09 | 2,57 | 1,16 | | | | o | |
| F1P8Z5 | Apo B | 87 | 0,68 | 19,3 | 0,08 | | x | | o | o |
| E2RNM1 | Apo D | 11,7 | 0,74 | 108,2 | 30,31 | | | | o | x |
| **intracellular proteins** | | | | | | | | | | |
| F1PZK8 | Dynein heavy chain domain 1 | DIV/0 | 182,61 | 3,24 | 1,35 | | | | x | | Ø |
| E2RB62 | Myosin IB | 6,19 | 0,23 | n.f. | | | | | o | |
| E2R1Q3 | Myosin XVIIIB | n.a. | | 3,02 | DIV/0 | | | | | x |
| J9P187 | Myosin IXA | 894 | 66,05 | 328 | 77,51 | | | | x | x | Ø |
| J9P007 | Kinesin-like protein | 3,43 | DIV/0 | 3,46 | 1,01 | | | | x | |

| | | | | | | | | | | | |
|---|---|---|---|---|---|---|---|---|---|---|---|
| F1PM26 | CD109 molecule | 17,3 | 6,15 | n.f. | | | | x | | | |
| J9P216 | Formin 1 | DIV/0 | 204,62 | 4,95 | DIV/0 | | | x | o | ⊘ | ● |
| J9P7F7 | Ficolin 1 | DIV/0 | 22,66 | 4,1 | 0,12 | | x | x | | | ⊘ |
| F1PAQ3 | Maltase-glucoamylase | 6,49 | 0,70 | 2,18 | 0 | | | o | | | |
| J9P5V6 | Talin 1 | 17,6 | 1,19 | 128 | 7,90 | | | o | x | ⊘ | ⊘ |
| E2RFS6 | Sacsin molecular chaperone | 12 | 2,87 | 12,7 | 2,54 | | | x | x | | |
| E2R926 | Fukutin | 29,5 | 3,72 | n.f. | | | | x | | ⊘ | |
| F1PRY8 | Dispatched RND transporter family member 2 | n.a. | | DIV/0 | DIV/0 | | | | x | | |
| F6Y1C9 | DNA Polymerase | n.a. | | 4,14 | 4,80 | | | | x | | |
| A0A0A0MPC5 | Trafficking protein particle complex subunit 2 | n.f. | | DIV/0 | DIV/0 | | | | x | | |
| J9P514 | RB associated KRAB zinc finger | 6,05 | 0,26 | 9,04 | DIV/0 | | | o | x | | |
| J9P653 | Ciliogenesis-associated TTC17-interacting protein (hum. homolog) | 10,1 | DIV/0 | 2,9 | 0,16 | | | x | | | |
| E2R1C3 | Proteasome subunit alpha type | 0 | 0 | 2,27 | 1,00 | | | | | | |
| J9NYS3 | Regulating synaptic membrane exocytosis 2 | 2,18 | 1,00 | 0 | 0 | | | | | | |
| E2RF52 | Proteasome subunit alpha type | n.f. | | 171 | DIV/0 | | | | x | | |
| **extracellular matrix proteins** | | | | | | | | | | | |
| J9P8M2 | Fibronectin | 28,9 | 0,46 | 32,6 | 0,11 | x | x | o | o | | ⊘ |
| J9NV47 | Hyaluronan binding protein 2 | 31,6 | DIV/0 | 2,18 | 0,20 | | x | x | | ⊘ | ● |
| F1Q3I5 | Collagen α1(I) chain | 2,87 | DIV/0 | 0 | 0 | x | | x | | ⊘ | |
| E5G723 | Collagen α1 (I) (Fragment) | 31,05 | 1,49 | DIV/0 | 137,80 | | | o | x | ● | ● |
| F1PHY1 | Collagen α2 (I) chain | 3,26 | 9,11 | 0 | 0 | | | x | | | |
| F1PJH3 | Collagen type XIII alpha 1 chain | 455 | DIV/0 | 0 | 0 | | | x | | ● | ● |
| J9NRV7 | Fibrinogen α-chain | 265 | 9,74 | 0 | 0 | | | x | | | |
| F1PW65 | Fibrinogen β-chain | 96,9 | 3,28 | 37,4 | 0,42 | | | x | o | | |
| F1P8G0 | Fibrinogen γ-chain | 399 | 22,27 | 53,4 | 0,62 | | | x | o | | ● |
| H9GWY3 | Inter-alpha-trypsin inhibitor heavy chain 4 | 59,9 | 1,78 | 5,51 | 0,08 | | x | o | o | | ⊘ |
| J9NUI6 | Alpha-1-microglobulin/bikunin precursor | 33,6 | 0,20 | 75 | 1,24 | x | x | o | o | | |
| E2RQF8 | Extracellular matrix protein 1 (human homolog) | 24,1 | 1,88 | 0 | 0 | x | x | o | | | |

| | | | | | | | | | | | | |
|---|---|---|---|---|---|---|---|---|---|---|---|---|
| J9P669 | Cadherin 5 | 3,3 | 0,30 | | 5,54 | 1,45 | | | | o | | |
| J9PAA4 | Carboxypeptidase N subunit 2 | 5,81 | 0,72 | | 177 | 0,19 | | x | | o | o | |
| F1Q1P0 | Coiled-coil domain containing 178 | 39,7 | 22,95 | | 17,2 | 13,97 | | | | x | x | |
| E2R416 | Lumican | 44,5 | 2,11 | | 11,9 | 0,30 | | | | x | o | Ø ● |
| F6Y120 | Serine/arginine repetitive matrix protein 3 (homolog) | 20,90 | 9,22 | | 3,78 | 0,45 | | | | x | | |
| Q2YF02 | Vitronectin (fragment), | 68,7 | 14,64 | | 2,38 | 0,44 | | | | x | | Ø ● |
| H2BF45 | Attractin (fragment) | n.a. | | | 4,14 | 0 | | | | | | |
| F1Q117 | Attractin | n.a. | | | 2,75 | 0 | | | | | | |

Legend to Table 1:

\* The protein IDs refer to the UniProt database of ~47,800 canine entries (date: October 26, 2017).

Criteria defining a biomarker:

Biomarker selection in mice, see Muckova *et al.,* 2015.

BM candidates in dogs were selected from all identified protein chains in all analyzed 2D-sub-fractions after comparison of data from affected and non-affected dogs. Peptides supporting identified proteins in every 2D-sub-fractions from AM were compared with those of the homologeous 2D-sub-fraction from NM by Sieve©, as well as those from CF with those from NF. The quantifier "ratio" is related to the concentration quotient (AM/NM, CF/NF) and "hits" to the amount of the respective protein in the compared sub-fractions. Sieve data on each identified protein were sorted to our separation matrix by in-house macros to visualize the chromatographic distribution of altered protein variants according. Depending on the actual chromatographic distribution, a BM was defined either by a >2-fold higher concentration given by the "overall trend" (Formula 1; Table 1, columns 4 and 6) or by the mean of all ratios, when exclusive synergistic alterations were seen. Alternatively a >5-fold higher mean of ratios in distinct clusters of sub-fractions, i.e. a distinct strongly elevated variant (Table 1, columns 3 and 5) was used defining a BM as a fraction out of the entire protein family.

$$\text{Overall trend} = (M_i * H_i)/(H_d / M_d) \qquad \qquad \text{Formula 1}$$

$M_i$: mean of all increased ratios
$H_i$: counts of all increased hits
$M_d$: mean of all decreased ratios
$H_d$: counts of all decreased hits

Altogether, 118 protein chains indicating 103 proteins were higher in affected compared with non-affected dogs (males and females), and 79 proteins fulfilled the criteria of a BM candidate defined above,

n.a., not altered; n.f., not found; 0, no cluster in this direction; x, overall trend higher in AM and/or CF; o, increased parts/protein variants higher in AM and/or CF. The term "DIV/0" (division by zero, i.e. no peptides or hits were found in the control sample) indicates extraordinary higher values in affected animals. Mean ratio of all ratios of all sub-fractions with synergistic alteration of the protein (AM/NM or CF/NF).

Raw spectra were analyzed with Proteome Discoverer 1.3 and Sequest database search algorithm applying a false discovery rate of <0.5% and the UniProt database of -47,800 canine entries (date: 10/26/2017).

In the following, we show after receiver operating characteristic (ROC) analysis that the areas under the ROC curves (AUC) were well above 0.700 for ColXIII, HaBP2, and C4BPA and combinations therefrom. They also exhibit reliable quality characteristics (high sensitivity, low false positive rate), either alone or in combinations (Tables 2 to 9). This holds true for the entire study group as well as in separately analyzed patient sub-groups with higher values. Under inflammation (post-infectious glomerulopathy and vasculitis) acute phase protein (CRP, AGT, FGG) and complement components CFI and CFH additionally and expectedly produce high AUC, sensitivity, and lowest false positive rate.

*Table 2*     ROC analysis, comparison of values from entire patients with controls

| BM | Cut-off | Sensitivity | 1-specificity | AUC | BM*BM | Cut-off | Sensitivity | 1-specificity | AUC |
|---|---|---|---|---|---|---|---|---|---|
| | | | | | urine | | | | |
| ColXIII | 0.02 | 0.837 | 0.38 | 0.703 | ADP*C4BPA | 25.09 | 0.396 | 0.131 | 0.667 |
| HABP2 | 0.25 | 0.586 | 0.224 | 0.757 | ColXIII*FMN | 0.23 | 0.766 | 0.343 | 0.702 |
| C4BPA | 5.01 | 0.558 | 0.17 | 0.719 | ColXIII*C4BPA | 0.001 | 0.819 | 0.388 | 0.741 |
| CRP | 2.52 | 0.516 | 0.253 | 0.626 | HABP2*TGF-ß1 | 0.10 | 0.65 | 0.2 | 0.696 |
| CFH | 4.29 | 0.671 | 0.455 | 0.676 | HABP2*FGG | 143.4 | 0.595 | 0.255 | 0.714 |
| CFI | 0.70 | 0.516 | 0.354 | 0.605 | ColXIII*HABP2*C4BPA | 0.08 | 0.552 | 0.156 | 0.721 |

*Table 3*     ROC analysis, comparison of values from patients with obesity, hypertension, metabolic syndrome, and T2DM with controls

| BM | Cut-off | Sensitivity | 1-specificity | AUC | BM*BM | Cut-off | Sensitivity | 1-specificity | AUC |
|---|---|---|---|---|---|---|---|---|---|
| | | | | | urine | | | | |
| ColXIII | 0.18 | 0.600 | 0.300 | 0.717 | ColXIII*FMN | 8.07 | 0.667 | 0.253 | 0.771 |
| HABP2 | 0.19 | 0.615 | 0.306 | 0.726 | C4BP*ColXIII | 0.35 | 0.800 | 0.265 | 0.763 |
| CRP | 5.73 | 0.714 | 0.158 | 0.810 | HABP2*FGG | 212.9 | 0.462 | 0.173 | 0.736 |
| | | | | | C4BP*ColXIII*HABP3 | 0.10 | 0.636 | 0.156 | 0.766 |

*Table 4*  ROC analysis, comparison of values from patients with T1DM with controls

| BM | Cut-off | Sensitivity | 1-specificity | AUC | BM*BM | Cut-off | Sensitivity | 1-specificity | AUC |
|---|---|---|---|---|---|---|---|---|---|
| | | | | urine | | | | | |
| TGF-ß1 | 13.69 | 0.659 | 0.293 | 0.652 | ADP*PICP | 44.04 | 0.651 | 0.242 | 0.718 |
| CRP | 2.52 | 0.516 | 0.253 | 0.626 | HABP2*TGF-ß1 | 2.26 | 0.756 | 0.167 | 0.801 |
| CFH | 4.29 | 0.671 | 0.455 | 0.676 | | | | | |
| CFI | 0.70 | 0.516 | 0.354 | 0.605 | | | | | |

*Table 5*  ROC analysis, comparison of the sub-group of patients with renal agenesis, hypoplasia. multicystic renal dysplasia with all controls

| BM | Cut-off | Sensitivity | 1-specificity | AUC | BM*BM | Cut-off | Sensitivity | 1-specificity | AUC |
|---|---|---|---|---|---|---|---|---|---|
| | | | | urine | | | | | |
| ColXIII | 0.18 | 0.433 | 0.3 | 0.625 | ColXIII*C4BPA | 0.7664 | 0.536 | 0.224 | 0.696 |
| HABP2 | 0.185 | 0.778 | 0.306 | 0.714 | | | | | |
| C4BPA | 5.53 | 0.515 | 0.16 | 0.685 | | | | | |

*Table 6*  ROC analysis, comparison of the sub-group of patients with anomalies of the urinary tract, duplex kidneys with all controls

| BM | Cut-off | Sensitivity | 1-specificity | AUC | BM*BM | Cut-off | Sensitivity | 1-specificity | AUC |
|---|---|---|---|---|---|---|---|---|---|
| | | | | urine | | | | | |
| ColXIII | 0.18 | 0.611 | 0.300 | 0.692 | C4BP*ColXIII*HABP2 | 0.55 | 0.462 | 0.115 | 0.661 |
| HABP2 | 0.19 | 0.714 | 0.306 | 0.758 | | | | | |

*Table 7*        ROC analysis, comparison of the sub-group of patients with ADPKD and ARDKD with all controls

| BM | Cut-off | Sensitivity | 1-specificity | AUC | BM*BM | Cut-off | Sensitivity | 1-specificity | AUC |
|---|---|---|---|---|---|---|---|---|---|
| | | | | | **urine** | | | | |
| ColXIII | 0.07 | 0.857 | 0.36 | 0.717 | ADP*PICP | 260.4 | 0.500 | 0.061 | 0.727 |
| HABP2 | 0.29 | 0.636 | 0.194 | 0.744 | AGT*GS | 115.3 | 0.500 | 0.146 | 0.754 |
| C4BP | 3.46 | 0.692 | 0.240 | 0.693 | AGT*C9 | 245.8 | 0.600 | 0.073 | 0.754 |
| CRP | 7.54 | 0.529 | 0.149 | 0.656 | C4BP*ColXIII | 0.53 | 0.667 | 0.235 | 0.750 |
| CFH | 8.89 | 0.667 | 0.313 | 0.697 | GS*CFH | 624.0 | 0.700 | 0.253 | 0.754 |
| CFI | 1.70 | 0.462 | 0.131 | 0.669 | GS*C9 | 19728 | 0.600 | 0.091 | 0.754 |
| | | | | | GS*CFI | 184.1 | 0.700 | 0.111 | 0.796 |
| | | | | | HABP2*TGF-ß1 | 3.28 | 0.700 | 0.133 | 0.755 |
| | | | | | HABP2*FGG | 365.4 | 0.700 | 0.092 | 0.812 |
| | | | | | VTN*CFI | 4.83 | 0.667 | 0.101 | 0.765 |
| | | | | | CFH*CFI | 27.3 | 0.636 | 0.152 | 0.762 |
| | | | | | CFI*FGG | 2883 | 0.583 | 0.040 | 0.755 |
| | | | | | CFH*FGG | 9180 | 0.727 | 0.232 | 0.771 |
| | | | | | C4BP*ColXIII*HABP2 | 0.11 | 0.700 | 0.156 | 0.784 |

*Table 8*    ROC analysis, comparison of the sub-group of patients with post-infectious glomerulopathy and vasculitis (IgAN, HSP) with all controls

| BM | Cut-off | Sensitivity | 1-specificity | AUC | BM*BM | Cut-off | Sensitivity | 1-specificity | AUC |
|---|---|---|---|---|---|---|---|---|---|
| | | | | | urine | | | | |
| AGT | 1.37 | 0.857 | 0.125 | 0.894 | ADP*C4BP | 26.9 | 0.714 | 0.121 | 0.870 |
| ColXIII | 0.97 | 0.625 | 0.130 | 0.854 | AGT*GS | 220.5 | 0.667 | 0.052 | 0.872 |
| GS_1 | 155.5 | 0.667 | 0.071 | 0.815 | C4BP*ColXIII | 7.00 | 0.750 | 0.092 | 0.913 |
| LRGP1 | 1.97 | 0.625 | 0.158 | 0.746 | ColXIII*FMN | 70.9 | 0.625 | 0.101 | 0.888 |
| VTN | 3.27 | 0.500 | 0.158 | 0.730 | GS*CFH | 3601 | 0.667 | 0.111 | 0.840 |
| C4BP | 6.09 | 0.750 | 0.150 | 0.881 | GS*VTN | 380.2 | 0.667 | 0.081 | 0.847 |
| CRP | 4.08 | 0.750 | 0.208 | 0.867 | GS*CFI | 670.3 | 0.667 | 0.010 | 0.867 |
| CFH | 23.73 | 0.667 | 0.152 | 0.813 | HABP2*FGG | 1103 | 0.667 | 0.061 | 0.854 |
| CFI | 1.49 | 0.857 | 0.152 | 0.883 | VTN*CFI | 2.80 | 0.857 | 0.202 | 0.875 |
| FMN | 91.2 | 0.625 | 0.150 | 0.817 | AGT*VTN | 11.7 | 0.667 | 0.010 | 0.845 |
| FGG | 1408 | 0.875 | 0.178 | 0.906 | CFH*CFI | 116.1 | 0.667 | 0.071 | 0.875 |
| | | | | | CFH*FGG | 57539 | 0.667 | 0.061 | 0.862 |
| | | | | | CFI*FGG | 2152 | 0.857 | 0.101 | 0.921 |
| | | | | | CFI*a1AGP | 43.3 | 0.714 | 0.082 | 0.875 |
| | | | | | C4BP*ColXIII*HABP2 | 6.90 | 0.667 | 0.031 | 0.936 |

*Table 9*    ROC analysis, comparison of the sub-group of patients with AS, thin basement membrane nephropathy, and familiar hematuria with all controls

urine

| BM | Cut-off | Sensitivity | 1-specificity | AUC | BM*BM | Cut-off | Sensitivity | 1-specificity | AUC |
|---|---|---|---|---|---|---|---|---|---|
| ColXIII | 0.98 | 0.617 | 0.131 | 0.817 | ColXIII*FMN | 13.75 | 0.725 | 0.232 | 0.796 |
| HABP2 | 0.62 | 0.596 | 0.072 | 0.808 | C4BP*ColXIII | 5.20 | 0.723 | 0.102 | 0.856 |
| C4BP | 4.04 | 0.894 | 0.192 | 0.898 | GSCFH | 579.6 | 0.745 | 0.253 | 0.815 |
| CRP | 4.29 | 0.674 | 0.210 | 0.765 | LRGP*C1q | 0.90 | 0.667 | 0.105 | 0.801 |
| CFH | 21.72 | 0.851 | 0.153 | 0.903 | C9*CFH | 3058 | 0.617 | 0.121 | 0.799 |
| CFI | 1.07 | 0.596 | 0.224 | 0.718 | CRP*C1q | 0.72 | 0.694 | 0.221 | 0.806 |
| FGG | 183 | 0.591 | 0.1 | 0.753 | CFH*CFI | 20.74 | 0.702 | 0.182 | 0.850 |
| | | | | | C4BP*ColXIII*HABP2 | 2.16 | 0.652 | 0.052 | 0.819 |

In the following we show a plurality of significant differences between biomarker connections of patients and controls (Table 10) as well as combinations of biomarker concentrations (Table 11).

*Table 10*    Significance levels (p) of BM concentrations in urine elevated in patients compared to healthy controls.

| Parameter | concentration range | obesity, metabolic syndrome, hypertension, T2DM | T1DM | renal agenesis, multicystic renal dysplasia | anomalies of the urinary tract, duplex kidneys | ADPKD, ARPKD | nephronophtisis | post infectious GN, vasculitis (IgAN, HSP) | AS (autosomal or no genetics), TBMN, benign fam. hematuria | XLAS, less severe | XLAS, intermediate | XLAS, severe |
|---|---|---|---|---|---|---|---|---|---|---|---|---|
| n of patients (controls: 86-101) | | 10-138 | 40-44 | 25-33 | 9-18 | 10-16 | 3 | 6-8 | 10-13 | 8 | 14-16 | 8-11 |
| ADP | ng/mg c | 0.013 | | | | | | | | <0.001 | <.001 | 0.016 |
| a1AGP | ng/mg c | | | | | 0.064 | | 0.003 | 0.093 | 0.093 | <0.001 | <0.001 |
| AGT | ng/mg c | | 0.050 | | | 0.042 | | <0.001 | 0.068 | | | |
| **ColXIII** | ng/mg c | 0.013 | 0.031 | 0.044 | 0.004 | 0.005 | | <0.001 | 0.015 | | <0.001 | <0.001 |
| GS | ng/mg c | | | | | 0.016 | | 0.007 | | | | |
| LRGP1 | ng/mg c | | | | | | | 0.019 | | 0.012 | <0.001 | <0.001 |
| **HABP2** | ng/mg c | 0.006 | <0.001 | <0.001 | <0.001 | 0.012 | | 0.020 | 0.001 | 0.010 | <0.001 | <0.001 |
| PICP | pg/mg c | <0.001 | | | | 0.003 | | 0.002 | 0.003 | 0.007 | 0.072 | |
| TGF-ß1 | pg/mg c | 0.002 | | | | | | | | | | |
| VTN | ng/mg c | | | 0.046 | | 0.003 | | 0.029 | | | | |
| C9 | pg/mg c | | | | | | 0.053 | | | 0.016 | | |
| **C4BP** | ng/mg c | 0.009 | | 0.002 | 0.015 | 0.028 | | <0.001 | <0.001 | 0.037 | <0.001 | <0.001 |
| CRP | pg/mg c | <0.001 | | | | 0.065 | | <0.001 | 0.062 | <0.001 | <0.001 | 0.004 |
| CFH | ng/mg c | | | 0.045 | | 0.011 | | 0.006 | 0.033 | <0.001 | <0.001 | <0.001 |
| CFI | ng/mg c | | | | | 0.020 | | <0.001 | 0.098 | | <0.001 | <0.001 |
| FMN | pg/mg c | | | | | | | 0.002 | 0.013 | 0.057 | <0.001 | 0.002 |

| | | | | | | | | | | | | |
|---|---|---|---|---|---|---|---|---|---|---|---|---|
| LUM | ng/mg c | | | | | | | | | | | |
| FGG | ng/mg c | | | | | 0.014 | | <0.001 | 0.001 | 0.001 | <0.001 | |
| C1q | ng/mg c | | | 0.029 | 0.035 | | | | | | <0.001 | <0.001 |

n: counts of patient values (min-max) included. P< 0.05 (grey) is considered significant, p< 0.01 (dark grey) highly significant, U-test (Mann-Whitney). All other comparisons yield insignificant differences. Some p-values <0.10 are included to show tendency. Bold: favorited BMs. Concentrations are normalized by creatinine (c).

EP 4 397 976 A1

**Table 11** Significance levels (p) of combined BM concentrations in urine elevated in patients compared to healthy controls.

| product of BMs in urine | obesity, metabolic syndrome, hypertension, T2DM | T1DM | renal agenesis, multicystic renal dysplasia | anormalies of the urinary tract, duplex kidneys | ADPKD, ARPKD | nephronophtisis | post infectious GN, vasculitis (IgAN, HSP) | AS (autosomal or no genetics), TBMN, benign fam. hematuria | XLAS, less severe | XLAS, intermediate | XLAS, severe |
|---|---|---|---|---|---|---|---|---|---|---|---|
| n of patients (controls: 84-99) | 10-13 | 41-43 | 25-30 | 9-17 | 10-13 | 3 | 5-8 | 5-10 | 8 | 14-16 | 7-11 |
| ADP x PICP | | <0.001 | | | 0.009 | | 0.006 | 0.012 | <0.001 | <0.001 | 0.094 |
| ADP x C9 | | 0.011 | | | | | | | <0.001 | 0.003 | |
| ADP x C4BP | 0.03 | | 0.004 | 0.044 | 0.068 | 0.026 | <0.001 | 0<.001 | | 0.04 | <0.001 |
| AGT x GS | | | | | 0.007 | | 0.001 | | | | |
| AGT x C9 | | 0.019 | | | 0.007 | | | | | | |
| **ColXIII x C4BP** | 0.002 | 0.005 | <0.001 | 0.01 | 0.002 | | <0.001 | 0.002 | 0.076 | <0.001 | <0.001 |
| **ColXIII x FMN** | 0.002 | 0.005 | 0.014 | 0.021 | 0.021 | | <0.001 | 0.002 | 0.052 | <0.001 | <0.001 |
| GS x CFH | | | 0.056 | | 0.006 | | 0.002 | 0.068 | <0.001 | <0.001 | <0.001 |

| | 55 | 50 | 45 | 40 | 35 | 30 | 25 | 20 | 15 | 10 | 5 |
|---|---|---|---|---|---|---|---|---|---|---|---|
| GS x C9 | | | | | 0.007 | | | | 0.098 | | |
| GS x VTN | | | | | 0.008 | | 0.003 | | | | |
| GS x CFI | | | | | 0.001 | | 0.001 | | | 0.033 | 0.005 |
| LRGP1 x C1q | | | 0.025 | 0.059 | | | 0.033 | | 0.004 | <0.001 | <0.001 |
| **HABP2 x TGF-ß1** | 0.024 | <0.001 | 0.056 | 0.002 | 0.005 | | | | 0.031 | | 0.030 |
| **HABP2 x FGG** | 0.007 | <0.001 | 0.006 | 0.084 | <0.001 | | 0.001 | <0.001 | 0.004 | 0.001 | |
| VTN x AGT | | 0.05 | 0.012 | | 0.035 | | 0.003 | | | | |
| VTN x C9 | | 0.025 | | | | | | | | | |
| VTN x CFI | | | | | 0.002 | | <0.001 | | | | 0.019 |
| C9 x CFH | 0.082 | | | 0.099 | 0.024 | | | | 0.008 | <0.001 | <0.001 |
| CRP x C1q | 0.008 | | | 0.021 | | | 0.002 | | <0.001 | <0.001 | 0.001 |
| CFH x CFI | | | | 0.068 | 0.003 | | <0.001 | 0.029 | <0.001 | <0.001 | <0.001 |
| CFH x FGG | | | 0.096 | | 0.002 | | <0.001 | 0.008 | <0.001 | <0.001 | 0.056 |
| CFI x FGG | | | | | 0.003 | | <0.001 | 0.038 | 0.046 | <0.001 | |
| CFI x a1AGP | | | | | 0.035 | | <0.001 | | | <0.001 | <0.001 |
| CFI x GS | | | | | 0.053 | | 0.026 | | | 0.033 | 0.005 |
| FMN x FGG | | | | | 0.017 | | <0.001 | 0.008 | 0.005 | <0.001 | |
| **ColXIII x C4BP x HABP2** | <0.001 | 0.006 | 0.016 | 0.015 | <0.001 | | <0.001 | 0.003 | 0.012 | <0.001 | <0.001 |

n: counts of patient values (min-max) included. Measures of BM concentrations as in Table 10. P< 0.05 (grey) is considered significant, p< 0.01 (dark grey) highly significant, U-test (Mann-Whitney). All other comparisons yield insignificant differences. Some p-values <0.10 are included to show tendency. Bold: favorited BMs.

The following examples and drawings illustrate the present invention without, however, limiting the same thereto.

BRIEF DESCRIPTION OF THE DRAWINGS

**Figure 1.** *Time course of disease evolution of kidney diseases and biomarker concentrations.*

There are several hypothetical biomarker (BM) types, which are differently targeted by our approach.

A: pre-clinical applicable BM; its increase indicates alterations starting in a pre-clinical stage and persisting under progression.
B: true pre-clinical BM; its increase indicates only pre-clinical alterations.
C: early BM; its increase indicates alterations simultaneously with the development of early clinical signs, often overseen or neglected (e.g. in AS micro-hematuria).
D: late BM; its increase indicates subsequent alterations in manifested disease. This type (e.g. in AS (micro)-albuminuria, decline of eGFR) is usually already applied. This type was not subject of the present invention.

Type A and B were searched for in juvenile AS-mice (4 weeks, see Muckova *et al.,* 2015) and AS-dogs (7 weeks). This search might also detect type C.
The evaluation applying human samples comprising various points of individual disease development might loss type B. However, all detected and validated BM, type A, B, and/or C should be suitable for very early diagnosis of kidney injury.
For the inverse concentration course, i.e. declining concentrations under alteration the same holds true accordingly.

**Figure 2.** shows boxplots of single concentrations of preferred biomarker candidates determined by ELISA. Left panel: Samples from patients with X-linked Alport syndrome of different severity; right panel: other nephropathies. The order of groups is identical in all three charts of each panel and indicated in the bottom. Complete results (p-values) are given in Table 10, all other cases are not significant.

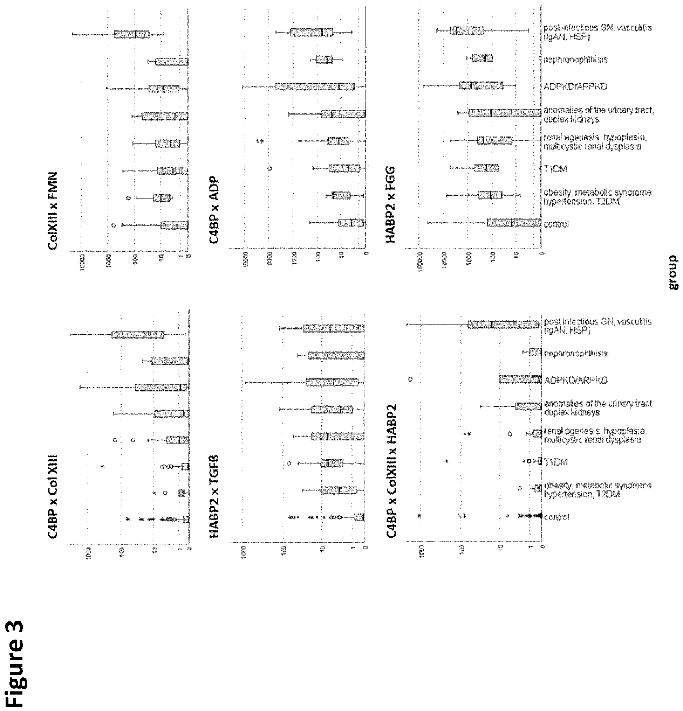

**Figure 3.** shows boxplots of preferred combined concentrations of biomarker candidates derived from ELISA with a cohort of patients and controls. Complete results (p-values) are given in Table 11. The order of groups is identical in all charts and is indicated in the bottom. The groups that are significantly different as determined by group wise comparison (P-values below 0.05 in post hoc analysis) are indicated by grey filling in Tables 10 and 11, all other cases are not significant.

EXAMPLES

EXAMPLE 1 Materials and Methods

**1.1 Patients and samples**

**Human samples:**

Two sets of patient samples have been applied: 1) real clinical samples derived from Alport patients (Tables 12) prospective samples from pediatric patients suffering from AS, type 1 diabetes (DM1) and various other nephropathies as well as from individuals with no known nephropathy as control group (Table 13). The first set comprises longitudinal serum and urine samples during early stages of AS progression. The second set was produced applying standard operation procedures to provide samples from healthy controls and patients with nephropathies in early stages to assess the sensitivity and specificity of our biomarker candidates for the aimed screening (ethic vote UHJ: 2020-1800). The informed consent of all participating subjects was obtained. All samples have been stored at -80°C until use.

Table 12 Data on samples from 35 male X-linked Alport patients

| sampling | Age (year, mean ± SD) | Age (year, range) | ACI therapy (n) | Serum creatinine (mg/dL, mean ± SD) | Urine albumin (mg/g creatinine, mean ± SD) | values |
|---|---|---|---|---|---|---|
| **all individuals** (n=35) | | | | | | **n** |
| First | 8.9 ± 4.1 | 3 - 17 | 20 | 0.44 ± 0.15 | 293.0 ± 601.9 | 32 |
| Last | 12.5 ± 4.1 | 6-21 | 24 | 0.58 ± 0.19 | 578.9 ± 1107.7 | 33 |
| **genetic variants** | | | | | | |
| less severe (n=7) | | | | | | |
| First | 10.3 ± 3.7 | 4 - 17 | 5 | 0.51 ± 0.10 | 16.2 ± 7.3 | 7 |
| Last | 13.6 ± 3.8 | 7-20 | 5 | 0.58 ± 0.13 | 14.8 ± 11.8 | 6 |
| intermediate (n=17) | | | | | | |
| First | 9.5 ± 4.3 | 3 - 17 | 6 | 0.43 ± 0.13 | 390.3 ± 789.8 | 15 |
| Last | 13.4 ± 4.3 | 6-21 | 10 | 0.62 ± 0.22 | 647.5 ± 1314.6 | 16 |
| severe (n=11) | | | | | | |
| First | 7.0 ± 3.0 | 3 - 13 | 9 | 0.38 ± 0.14 | 245.3 ± 391.8 | 10 |
| Last | 10.6 ± 3.0 | 7 - 17 | 9 | 0.51 ± 0.16 | 638.1 ± 683.9 | 11 |

The informed consent of all participating subjects was obtained. Inclusion criteria were age >3 years and clinical diagnosis of AS according to international criteria.

*Table 13* **Basic and clinical characteristics in the study population and controls.**

Data are presented as mean, (SD), and range. * P< 0.05 is considered significant, ** p< 0.01 highly significant, Mann-Whitney, U-test. Unmarked patient values are not significantly different to controls as well as values of female to male controls.

| group | n | gender | Age [y] | BMI [SDS, z] | eGFR [mL/min/1.73m²] | Cystatin CGFR [mL/min/1.73m²] | Cystatin C [serum] mg/L | Blood glucose [mM] | HBA1c [%] | Blood pressure syst. [SDS, z] | Blood pressure diast [SDS, z] | Serum creatinine [µM] | Urine albumin [mg/g creatinine] |
|---|---|---|---|---|---|---|---|---|---|---|---|---|---|
| controls | 48 | male | 10.3 (4.1, 3.1-17.1) | 0.220 (1.28, -2.50-3.68) | 145.6 (25.6) | 113.2 (23.1) | 0.87 (0.12) | 5.01 (0.60) | 5.19 (0.29) | 0.490 (0.997) | 0.274 (0.866) | 51.0 (15.8) | 6.9 (5.9) |
| controls | 56 | female | 10.3 (4.0, 3.5-16.7) | -0.064 (1.25, -3.44-2.90) | 153.4 (30.0) | 121.5 (22.5) | 0.81 (0.11) | 5.00 (1.88) | 5.17 (0.28) | 0.325 (1.00) | 0.409 (1.01) | 45.5 (12.7) | 11.1 (12.0) |
| **controls** | 104 | 48 m/56 f | 10.3 (4.0, 3.1-17.1) | 0.069 (1.264, -3.44-3.68) | 149.8 (28.2) | 117.7 (23.0) | 0.84 (0.12) | 5.01 (1.44) | 5.18 (0.28) | 0.404 (0.999) | 0.344 (0.943) | 48.1 (14.4) | 9.2 (9.9) |

| | | | | | | | | | | | | | |
|---|---|---|---|---|---|---|---|---|---|---|---|---|---|
| metabolic syndrome, hypertension, adipositas, T2DM | 18 | 7 m/11 f | 14.6** (2.8) | 2.55** (0.85, 0.58 - 4.34) | 154.8 (34.7) | 112.1 (20.1) | 0.83 (0.13) | 6.01** (1.29) | 5.50** (0.39) | 2.048** (1.27) | 1.981** (1.31) | 53.2 (10.2) | 5.3 (4.8) |
| T1DM | 52 | 27 m/25 f | 14.7** (4.1) | 0.629** (1.16, -2.96 - 3.23) | n. d. | 107.5* (19.3) | 0.87* (0.14) | 8.6** (3.8) | 7.68** (1.01) | 1.370** (1.10) | 1.456** (0.932) | 52.0 (19.0) | 16.4 (49.5) |
| renal agenesis, hypoplasia. multicystic renal dysplasia | 33 | 21 m/ 12 f | 11.4 (4.3) | 0.268 (1.197, -2.68 - 3.25) | 138.9 (27.4) | 101.3* (14.7) | 0.94** (0.11) | 5.44** (0.68) | 5.27 (0.27) | 1.452** (1.27) | 0.556 (0.837) | 54.4 (15.5) | 23.6 (47.3) |
| anomalies of the urinary tract, duplex kidneys | 19 | 11 m/8 f | 10.9 (3.8) | 0.438 (1.212, -2.68 - 3.25) | 141.5 (27.9) | 118.1 (28.7) | 0.85 (0.16) | 5.25** (0.48) | 5.23 (0.25) | 1.647** (0.856) | 0.577 (0.815) | 52.8 (14.1) | 17.9 (24.5) |
| ADPKD. ARPKD | 17 | 9 m/8 f | 10.4 (4.5) | 0.091 (0.930, -1.58 - 1.92 | 156.7 (29.0) | 117.1 (19.0) | 0.84 (0.12) | 5.25* (0.27) | 5.19 (0.25) | 1.587** (1.14) | 1.101 (0.975) | 45.9 (11.1) | 95.6** (205.5) |
| nephrono-phthisis | 3 | 1 m/2 f | 8.5 (1.6) | -0.270 0.488, -0.96 - 0.10) | 137.8 (32.8) | 99.3 (12.7) | 0.98* (0.11) | 5.20 (0.43) | 5.33 (0.25) | 1.707* (0.684) | 1.771** (0.441) | 46.7 (12.5) | 11.6 (5.7) |

| | | | | | | | | | | | | | |
|---|---|---|---|---|---|---|---|---|---|---|---|---|---|
| post-infectious GN, vasculitis (IgAN, HSP) | 9 | 3m/6 f | 13.7** (2.3) | 0.083 (1.746, -2.31 - 2.43) | 146.8 (29.7) | 100.8** (18.7) | 0.93** 0.14) | 5.87* (0.85) | 5.34 (0.35) | 1.001 (0.835) | 0.054 (0.754) | 55.0 (14.6) | 57.6* (65.3) |
| AS⁺ TBMN, benign fam. hematuria | 15 | 8 m/7 f | 12.8 (3.1) | 0.318 (1.131, -1.81 - 2.41) | 154.6 (22.9) | 117.6 (22.8) | 0.82 (0.11) | 5.14 (0.67) | 5.31* (0.20) | 0.658* (0.983) | 0.137 (0.536) | 52.6 (13.2) | 106.1** (285.2) |

⁺autosomal AS and histological approved AS without genetics;

Age- and sex- dependent standard values (SDS) of BMI according to Kromeyer-Hauschild *et al.,* 2001 ans SDS transformation of blood pressure vslues according to National High Blood Pressure Education Program Working Group (see Roccella *et al.,* 2004).

Healthy pediatric controls include 70 samples acquired from the Leipzig Medical Biobank (Leipzig Research Center for Civilisation Diseases. LIFE-child (collection: 02/2014-11/2014)) as well as 34 pediatric controls acquires in Jena (09/2020-09/2021).

The participants of the second set were recruited interviewed and examined from September 2020 to November 2021, see Table 14.

The following *standard operation procedure* was applied: Blood samples from individuals were obtained in the morning. Blood was drawn by venipuncture into 9 mL-monovettes (Sarstedt, Nümbrecht, Germany). To obtain serum, blood was collected into serum monovettes (02.1063.001). If not stated otherwise, the clotting time was 90 min at room temperature. All samples were centrifuged at 1500xg for 10 min at 20°C, immediately aliquoted and frozen at - 20°C not longer than 3 days before storage at -82°C until use. Plasma was obtained by drawing blood into EDTA-containing containers (02.1066.001). If not stated otherwise, samples were immediately centrifuged, aliquoted and stored as described above. Urine samples: Urine samples were obtained as spontaneous void urine close to venipuncture, aliquoted, and frozen at -20°C not longer than 3 days before storage at -82°C until use.

Thirtytwo healthy controls were enrolled among subjects without any signs and history of any known nephropathy or inflammatory signs. The samples from control individuals were obtained along with the second set. Moreover, 72 further healthy controls were acquired from an independent cohort of pediatric control samples from the Leipzig Medical Biobank (Leipzig Research Center for Civilization Diseases, LIFE-child), obtained in 2014.

### 1.2. Samples for proteomic search

### Animals

Four litters have been produced from a colony of mixed-breed dogs with X-linked AS (COL4A5-) dogs (Lees, 2013).

Genotyping was performed using PCR of DNA isolated from blood. All animals have been weaned at 6 weeks of age and raised with an established standard diet, husbandry, and socialization protocol.

### Sampling protocol

A minimum of 4 ml of whole blood for harvesting serum and EDTA-plasma and 3 ml urine (voided) were collected. Unaffected dogs were randomly paired with their affected littermates for the sampling schedule. A complete standard urinalysis, UPC, and urine albumin concentration have been determined. All samples were collected and processed by standard methods to minimize pre-analytical variation. A minimum of two 0.5 ml aliquots of each sample type was stored at -80°C for future analysis and shipped on dry ice.

### Samples used for proteomic search

Equal volumes of three plasma samples of each animal group (affected males (AM), carrier females (CF) in a pre-clinical stage (wk. 7), and unaffected normal siblings (NM, NF, wk. 7)) have been combined. By this, equal volumes and practically equal protein amounts of each pool have been pre-fractionated and subsequently analyzed (Table 14).

*Table 14* Data of pooled dog samples used for proteomic search

| Pool | Litter | Pooled EDTA-plasma samples | | | Laboratory data | | | |
|---|---|---|---|---|---|---|---|---|
| | | Input for chromatographic pre-fractionation | | | Urine | | Serum | |
| | | Vol. (ml) | Pro-tein (mg/ml) | Protein (mg) | UPC | Dipstick Blood | Crea-tinine mg/dl | Albumin g/dl |
| NM | 1, 2, 2 | 0.78 | 78.85 | 61.5 | 0.48±0.41 | Tr, N, N | 0.39±0.01 | 2.80±0.08 |
| NF | 1, 3, 3 | 0.78 | 80.97 | 63.2 | 0.36±0.34 | N, N, Tr | 0.38±0.05 | 2.50±0.14 |
| CF | 1, 3, 3 | 0.78 | 83.58 | 65.2 | 0.14±0.11 | N, N, 1+ | 0.36±0.03 | 2.50±0.00 |
| AM | 1, 3, 3 | 0.78 | 79.27 | 61.8 | 0.92±0.53 | 1+, 2+, Tr | 0.35±0.04 | 2.53±0.09 |
| Samples were obtained at week 7 of age; Tr: trace, N: negative. | | | | | | | | |

### 1.3. Proteomic search

EDTA-plasma pools of all four groups have been separated, prepared, and analyzed by mass spectrometry, throughout in parallel.

**Sample fractionation:** The inventors' method as described in Rhode *et al.* (2019) and Wendler *et al.* (2013) combines native size exclusion (SEC, first dimension, 1D), followed by anion exchange (AEC, 2D) chromatography. After serial 1D-fractionation, all further procedures are performed in microplate format. Thus, beginning with 2D-fractionation, parallelization and automation is achieved for separation, spectrophotometric readout, temporary storage, hit picking, medium exchange, digest, desalting, and finally application to the autosampler followed by LC-MS. From each sample 96 1D-fractions are produced. Each 1D-fraction is further separated into 43 2D-fractions. Thus, at least 4128 2D-sub-fractions are produced containing 20-40 different proteins with total protein concentrations ranging from zero to 4.3 mg/ml. Fractions of all samples exhibiting the same position (1D_2D) are called homologous fractions. Since the inventors' separation is highly reproducible, each sample constituent is expected to be distributed into an identical set of homologous fractions with all samples. Diseases may alter concentrations and/or posttranslational modification of a sample constituent. Both changes can be detected by altered concentration ratios of homologous fractions. Moreover, each single protein to be recovered is expected within a set of at least three consecutive fractions due to their chromatographic distribution. Due to huge time requirements for mass spectrometric analysis (see below) not all but a representative portion of fractions have been selected for analysis, i.e. every second fraction in all chromatographic spots exhibiting sufficient protein content (>0.03 mg/ml).

### 1.4. LC-MS/MS analysis

Separation of tryptic peptides prior to mass spectrometry was performed on a Hypersil Gold UHPLC column (1.9 $\mu$m, 50×1.0 mm) using an Accela 1250 UHPLC system (both Thermo Fisher Scientific, USA) using binary gradient elution of the mobile phase, i.e. 0.1% formic acid in (A) water and in (B) acetonitrile at a flow rate of 150 $\mu$L/min, throughout (0-1 min 5% B, 21 min 30% B, 24 min 40% B, 25 min 90% B, 25.1-26 min 90% B, 26.1-30 min 5% B). Tandem mass spectrometry (MS/MS) measurements were carried out on a LTQ Orbitrap Discovery (Thermo Fisher Scientific, USA) by positive heated electrospray ionization (H-ESI) at a vaporizer temperature of 200 °C. Sheath gas flow (30.0) and auxiliary gas flow (10.0) were used to dry the ionspray (both nitrogen gas flows in arbitrary units). The ionization voltage and the temperature of the ion transfer tube were set to 4.5 kV and to 275 °C, respectively. The MS/MS system operated in data-dependent TOP10 mode using 1 microscan. For this purpose, ions have been monitored in the LTQ ion trap in full scan centroid mode at m/z 350-1700. The ten most intensive ions run through collision-induced dissociation (CID) for further orbitrap high resolution (30,000) analysis (profile data type). Wideband activation was used. The automatic gain control (AGC) target value for the Orbitrap mass analyzer in full scan mode was $1.0 \times 106$. The LC-MS/MS operated via graphical interface of the Xcalibur software 2.1. Fractions were run in duplicates. After each duplicate, a blank (i.e., water sample) was applied to avoid carryover. For quality control and check a digest of 50 $\mu$g/mL transferrin (human (Holo), SERVA Electrophoresis GmbH, 36756) was analyzed four times per microplate.

### 1.5. Protein identification and data analysis

**Protein identification:** MS/MS spectra were analyzed using the Proteome Discoverer 1.3 and Sequest database search (repeatedly updated Human Fasta databases) downloaded from www.uniprot.org, 10/26/2017) applying a false discovery rate of 0.01.

**Pairwise analysis:** To detect differences between two samples (affected vs control, technical duplicates) analysis was performed by SIEVE 2.0 (Software for label-free differential analysis, Thermo Fisher Scientific, USA). MS-data (XCALIBUR-generated raw-files) were imported and analyzed as "Two sample differential analysis" in pairs of corresponding fractions. The results were filtered by the charge-dependent Xcorr (Xcorr 1.5, 2.0, 2.25 and 2.5 for charge 1, 2, 3, and > 3, respectively) and by a p-value of 0.05. The results are expressed in fold-change ratios between affected and control. Subsequent protein lists were exported to Excel and labeled with fraction locations (1D_2D). Thereafter, all proteins from all fractions were merged and sorted according to their Protein-ID followed by 1D- and 2D-location applying macros. Thus, we were able to identify clusters of peptides pertaining to distinct proteins with a similar ratio and direction. The process of clustering was adapted dynamically on the distribution of peptide counts: two to six consecutive 1D- and/or 2D-fractions were subsumed as a cluster. However, associated fractions with opposite ratios were not considered as clusters. Proteins with ratios >4 and <0.25 were regarded as stringently up- and down-regulated, respectively, when occurring in at least two locations irrespective of lower (synergistic) ratios in adjacent chromatographic fractions. Protein clusters were assigned to three categories: proteins with exclusively positive or negative ratios mirroring the alteration of the total concentration of the respective protein in the sample, and proteins with both up-and down-regulated clusters. With the latter proteins altered posttranslational modification could promote an altered distribution along chromatography. All categories are considered as indicative alterations. Proteins were finally selected in a single result list (see Table 1) based upon ratios and hit numbers.

## 1.6. Biomarker candidate verification

The protein clusters mentioned in section 1.5. represent distinct variants (proteoforms) of the respective protein family of different concentration in blood of affected but pre-clinical animals in comparison to the respective controls. The specific characteristics of these variants (i.e. PTM, complexes, and fragments) are not known until today as well as their tissue origin and the degree of their appearance in urine, the specimens of high practicability for (pediatric) screening purposes.

Thus, for pre-evaluation 27 biomarker candidates and variants therefrom were immune-quantified in serum, EDTA plasma, and urine from a small number of individuals of all groups of dogs (NM, AM, NF, CF) using another sample set than for proteomic search has been selected and commercially available ELISA-kits, cf. Table 15A.

Thereafter a stepwise procedure has been conducted in humans. Firstly, evaluation of 28 biomarker candidates was performed in 6 to 8 sets of characteristic blood and urine samples from individuals out of the patient and control group. All assays were performed in duplicates according to the manufacture's manuals. Secondly, promising biomarker candidates that show different mean values between patients and controls have been quantified in a maximum of samples of our biobank. The commercially available ELISA-kits applied are summarized in Table 15B mostly with prolonged incubation times to get the high sensitivity required.

**Analysis of ELISA results:** Data were analyzed by Sigma Plot software version 14.5. Firstly, normal distribution of data sets was analyzed by Shapiro-Wilk Test For data that did not follow normal distribution a Kruskal Wallis One Way ANOVA on ranks was used. Post hoc analysis was performed by pairwise (U-test). Statistical significance was set at $p<0.05$. Secondly, a correlation analysis according to Pearson and Spearman, by SPSS program (version 27), has been performed of immune reactivities of biomarker proteins among each other. Thereby positively and negatively correlated proteins could be identified and combined by multiplication.

*Table 15* Specification of ELISA-Test kits applied to dog and human samples

*Table 15A* Measurements in dog samples

| antigen | supplier | code | Species | function |
|---|---|---|---|---|
| kallikrein | | KTE20170 | dog | **n** |
| vitronectin | | KTE20161 | dog | **n** |
| dynein | | KTE20163 | dog | **n** |
| hyaluronan binding protein 2 | | KTE20164 | dog | **n** |
| fukutin | Abbkine | KTE20171 | dog | **n** |
| myosin IXA | | KTE20175 | dog | **n** |
| collagen 1 alpha 1 | | KTE20165 | dog | **n** |

(continued)

| antigen | supplier | code | Species | function |
|---|---|---|---|---|
| collagen 1 alpha 2 | | KTE20166 | dog | **n** |
| collagen 13 alpha 1 | | KTE20169 | dog | **n** |
| formin 1 | | KTE20173 | dog | **n** |
| talin-1 | CUSABIO | CSB-EL023597HU | man | **n** |
| serum amyloid A | Genorise | GR115278 | dog | **n** |
| leucin rich alpha-2-glycoprotein | | MBS075175 | dog | **y** |
| gelsolin | | MBS2603447 | dog | **y** |
| adiponectin | | MBS778758 | dog | **n** |
| adiponectin | | MBS080454 | cat | **y** |
| lumican | MyBioSource | MBS2607240 | dog | **y** |
| procollagen I C-terminal | | MBS2608196 | dog | **y** |
| talin-1 | | MBS2604628 | dog | **y** |
| serum amyloid | | MBS740421 | dog | **y** |
| serum amyloid | | MBS1602508 | dog | **y** |
| lumican | Cloud-Clone | USC-SEB496HU | man | **y** |
| collagen type 13 | | SEC138HU | man | **y** |
| vitronectin | RayBiotech: | ELH-VTN | man | **y** |
| adiponectin | | RD19102310 | man | **y** |
| TGF-B1 | Biovendor | BVD-RAF122R | man | **y** |
| adiponectin | | BVD- | man | **y** |

*Table 15B* Measurements in human samples

| antigen | supplier | code | Species | function |
|---|---|---|---|---|
| angiotensinogen | Abbexa | abx573778 | man | **y** |
| formin 1 | | abx387383 | man | y |
| alpha-1-acid glycoprotein | | HEA816Hu | man | y |
| coagulation factor XIIIB | | CEB613Hu | man | y |
| complement C1q | | SEA747Hu | man | y |
| complement factor H | | SEA635Hu | man | y |
| complement factor I | | SEB978Hu | man | y |
| collagen type XIII | | SEC138Hu | man | y |
| c-reactive protein | | SEA821Hu | man | y |
| fibronectin | Cloud- Clone | USC-HEA037HU | man | y |
| ficolin | | SEA786Hu | man | y |
| hemopexin | | SEB986HU | man | y |
| gelsolin | | SEA372Hu | man | y |
| C4 binding protein alpha | | SEB620Hu | man | y |
| retinol binding protein 4 | | SEA929Hu | man | y |
| talin | | SEA278Hu | man | y |
| leucine rich alpha-2- | | HEB934Hu high | man | y |
| lumican | | ELH-LLTM-1 | man | y |
| serum amyloid A | RayBiotech | ELH-SAA-1 | man | y |
| vitronectin | | (ELH-VTN | man | y |
| carboxyterminal propeptide of | | CSB-E08079h | man | y |
| fibrinogen gamma chain | | CSB-E13319h | man | y |
| alpha-trypsin inhibitor heavy | CUSABIO | CSB-E17022h | man | y |
| hyaluronan-binding protein 2 | | CSB-EL010113HU | man | y |
| complement component C9 | | CSB-E14011h | man | y |

EP 4 397 976 A1

(continued)

| antigen | supplier | code | Species | function |
|---|---|---|---|---|
| serum amyloid A | Boster | BOS-EK1544, | man | y |
| TGF-β1 | Biovendor | BVD-RAF122R* | man | y |
| adiponectin | | BVD-RD191023100* | man | y |

**A** Measurement in dog samples: When no dog-specific or reliable kit was available, we applied assays of other species-specificity due to the high sequence similarities. Out of the checked kits those marked by yes (y) produced reliable antigen concentrations. The other kits either were not able to detect the proteoform naturally occurring in our samples or were not sensitive enough.

**B** Measurement in human samples: The kits were performed according to the suppliers instructions with the following modifications: all assays from Cloud-Clone and CUSBIO were performed with doubled incubation times. Samples were pre-incubated with 0.5% Tween 20 before measured of gelsolin. For the quantification of PICP, TGF-β1, and CRP in urine either incubation times were prolonged or concentrated specimens were applied. For that samples have been concentrated in Amicon® Ultra -15 Centrifugal filters (3K, LTFC900324) by ~10 to ~60 fold depending on the starting volume available.

The features disclosed in the foregoing description, in the claims and/or in the accompanying drawings may, both separately and in any combination thereof, be material for realizing the invention in diverse forms thereof.

REFERENCES

Baum, A. et al. Searching biomarker candidates in serum using multidimensional native chromatography. II Method evaluation with Alport syndrome and severe inflammation. Journal of chromatography. B, Analytical technologies in the biomedical and life sciences 876, 31-40 (2008).

I. H. de Boer, M. L. Caramori, J. C. N. Chan, H. J. L. Heerspink, C. Hurst, K. Khunti, et al., Executive summary of the 2020 KDIGO Diabetes Management in CKD Guideline: evidence-based advances in monitoring and treatment, Kidney Int 98 (4): 839-848 (2020).

Dufek, B. et al. Endothelin A receptor activation on mesangial cells initiates Alport glomerular disease. Kidney international 90, 300-310 (2016).

Gessel, M.M. et al. Two Specific Sulfatide Species Are Dysregulated during Renal Development in a Mouse Model of Alport Syndrome. Lipids 54, 411-418 (2019).

Goka, S., Copelovitch, L. & Levy Erez, D. Long-term outcome among females with Alport syndrome from a single pediatric center. Pediatric nephrology 36, 945-951 (2021).

Grodecki, K.M. et al. Treatment of X-linked hereditary nephritis in Samoyed dogs with angiotensin converting enzyme (ACE) inhibitor. Journal of comparative pathology 117, 209-225 (1997).

Gross, O. et al. Preemptive ramipril therapy delays renal failure and reduces renal fibrosis in COL4A3-knockout mice with Alport syndrome. Kidney Int 63, 438-446 (2003).

Gross, O. et al. Safety and Efficacy of the ACE-Inhibitor Ramipril in Alport Syndrome: The Double-Blind, Randomized, Placebo-Controlled, Multicenter Phase III EARLY PROTECT Alport Trial in Pediatric Patients. ISRN pediatrics 2012, 436046 (2012)

Gross, O. et al. Advances and unmet needs in genetic, basic and clinical science in Alport syndrome: report from the 2015 International Workshop on Alport Syndrome. Nephrology, dialysis, transplantation: official publication of the European Dialysis and Transplant Association - European Renal Association 32, 916-924 (2017).

Guo, J. et al. Dysregulated Expression of microRNA-21 and Disease-Related Genes in Human Patients and in a Mouse Model of Alport Syndrome. Hum Gene Ther 30, 865-881 (2019).

Hertz, J.M., Thomassen, M., Storey, H. & Flinter, F. Clinical utility gene card for: Alport syndrome. Eur J Hum Genet 20 (2012).

Hudson, B.G., Tryggvason, K., Sundaramoorthy, M. & Neilson, E.G. Alport's syndrome, Goodpasture's syndrome, and type IV collagen. N Engl J Med 348, 2543-2556 (2003).

Jerums G, Panagiotopolous S, Premaratne E, MacIsaac RJ. Integrating albuminurea and GFR in the assessment of diabetic nephropathy. Nat Rev Nephrol 5(7), 397-406 (2009). Accession No. 19556994 DOI: 10.1038/nrneph.2009.91

Kashtan, C.E. Animal models of Alport syndrome. Nephrology, dialysis, transplantation: official publication of the European Dialysis and Transplant Association - European Renal Association 17, 1359-1362 (2002).

Kashtan, C.E. et al. Clinical practice recommendations for the treatment of Alport syndrome: a statement of the Alport Syndrome Research Collaborative. Pediatr Nephrol 28, 5-11 (2013).

Koopman, J.J.E., van Essen, M.F., Rennke, H.G., de Vries, A.P.J. & van Kooten, C. Deposition of the Membrane Attack Complex in Healthy and Diseased Human Kidneys. Front Immunol 11, 599974 (2020).

Kromeyer-Hauschild, K., Wabitsch, M., Kunze, D., Geller, F., Geiβ, H. C., Hesse, V., & Hebebrand, J. Monatsschr. Kinderheilk. 149 (2001).

Lees, G.E. Kidney diseases caused by glomerular basement membrane type IV collagen defects in dogs. J Vet Emerg Crit Care (San Antonio) 23, 184-193 (2013).

Muckova, P. et al. Preclinical Alterations in the Serum of COL(IV)A3(-)/(-) Mice as Early Biomarkers of Alport Syndrome. JProteome Res 14, 5202-5214 (2015).

Nagel, M., Nagorka, S. & Gross, O. Novel COL4A5, COL4A4, and COL4A3 mutations in Alport syndrome. Hum Mutat 26, 60 (2005).

Noone, D. & Licht, C. An update on the pathomechanisms and future therapies of Alport syndrome. Pediatr Nephrol 28, 1025-1036 (2013).

Pohl, M. et al. Diagnosis of Alport syndrome--search for proteomic biomarkers in body fluids. Pediatric nephrology 28, 2117-2123 (2013).

Rhode, H., Muckova, P., Buchler, R., Wendler, S., Tautkus, B., Vogel, M. et al. A next generation setup for pre-fractionation of non-denatured proteins reveals diverse albumin proteoforms each carrying several post-translational modifications. Sci Rep 2019 Vol. 9 Issue 1 Pages 11733. Accession Number: 31409882 DOI: 10.1038/s41598-019-48278-y.

Roccella EJ et al. (National High Blood Pressure Education Program Working Group on High Blood Pressure in Children and Adolescents). The fourth report on the diagnosis, evaluation, and treatment of high blood pressure in children and adolescents. Pediatrics. 114 (S2) 555-576 (2004).

Savige, J. et al. Expert consensus guidelines for the genetic diagnosis of Alport syndrome. Pediatr Nephrol (2018).

Savige, J. et al. Expert consensus guidelines for the genetic diagnosis of Alport syndrome. Pediatric nephrology 34, 1175-1189 (2019).

Wendler, S., Tautkus, B., Nemitz, S., Pesek, J., Krüger, T., Opitz, S., Bartz, M., Richter, S., Oehme, H., Haenel, T., Moore, T., Kreusch, S., Hanf, B., Schmidt, L., Rhode, H. in Automations Systems of the 21st Century. (ed. D. Arent, Freebush, M.) 1-50 (nova publishers, New York; 2013).

Yamamura, T. et al. Development of an exon skipping therapy for X-linked Alport syndrome with truncating variants in COL4A5. Nat Commun 11, 2777 (2020).

**Claims**

1. A *combination of at least two proteins* selected from

   Collagen type XIII (COLXIII),
   Hyaluronan-Binding Protein 2 (HABP2),
   C4 Binding Protein (C4BP),
   Complement Factor H (CFH), and
   Complement Factor I (CFI)

   for use as biomarker in a method for the pre-clinical and/or early-stage detection and/or diagnosis of a kidney disease in a mammal.

2. The combination for use according to claim 1, wherein

   COLXIII and C4BP, or
   COLXIII and C4BP and HABP2, or
   CFH and CFI are determined, or

   wherein COLXIII, HABP2, C4BP, CFH, and CFI are determined.

3. A *protein* selected from

52

Collagen type XIII (COLXIII),
Hyaluronan-Binding Protein 2 (HABP2),
C4 Binding Protein (C4BP),
Complement Factor H (CFH), and
Complement Factor I (CFI)

*in combination with at least one further protein* which is selected from:

Fibrinogen,
Lumican (LUM),
Formin 1 (FMN),
Vitronectin (VTN),
Gelsolin (GS),
Angiotensinogen (AGT),
Adiponectin (ADP),
Transforming growth factor-$\beta$1 (TGF-$\beta$1),
Carboxyterminal Propeptid of Type I Procollagen (PICP),
C Reactive Protein (CRP),
$\alpha$-1-Acid Glycoprotein (a1AGP),
Coagulationfactor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich $\alpha$-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK),
Myosin IXA (MYIXA),
Kininogen 1,
$\alpha$2-HS Glycoprotein,
Complement C6,
Complement C7,
Complement C8,
APOH, Beta-2-glycoprotein 1,
Serpin family A member 1,
Transferrin,
$\alpha$1-B Glycoprotein,
Apo AI,
Apo D,
Alpha-1-microglobulin/bikunin precursor,
Carboxypeptidase N subunit 2, and
Serine/arginine repetitive matrix protein 3,

said *at least one further protein* being preferably selected from

Fibrinogen,
Lumican (LUM),
Formin 1 (FMN),
Vitronectin (VTN),
Gelsolin (GS),
Angiotensinogen (AGT),
Adiponectin (ADP),

Transforming growth factor-β1 (TGF-β1),
Carboxyterminal Propeptid of Type I Procollagen (PICP), and
C Reactive Protein (CRP),

for use as biomarker in a method for the pre-clinical and/or early-stage detection and/or diagnosis of a kidney disease in a mammal.

4. The combination for use according to claim 3, wherein

      C4BP and ADP, or
      COLXIII and FMN, or
      HABP2 and TGF-β1, or
      HABP2 and FGG, or
      CFH and GS, or
      CFI and GS, or
      CFI and VTN, or
      CFH and FGG, or
      CFI and FGG

are determined.

5. A *combination of at least two proteins* selected from

      Collagen type XIII (COLXIII),
      Hyaluronan-Binding Protein 2 (HABP2),
      C4 Binding Protein (C4BP),
      Complement Factor H (CFH),
      Complement Factor I (CFI),
      Fibrinogen,
      Lumican (LUM),
      Formin 1 (FMN),
      Vitronectin (VTN),
      Gelsolin (GS),
      Angiotensinogen (AGT),
      Adiponectin (ADP),
      Transforming growth factor-β1 (TGF-β1),
      Carboxyterminal Propeptid of Type I Procollagen (PICP),
      C Reactive Protein (CRP),
      α-1-Acid Glycoprotein (alAGP),
      Coagulationfactor XIII (CFXIII),
      Complement Component 1q (C1q),
      Complement Component 9 (C9),
      Fibronectin (FN),
      Ficolin 1 (FIC),
      Hemopexin (HPX),
      Inter-alpha-trypsin inhibitor 4 (IATI4),
      Leucine Rich α-2-Glycoprotein 1 (LRGP1),
      Retinol-Binding Protein 4 (RBP4),
      Serum Amyloid A (SAA),
      Talin 1 (Tal),
      Kallikrein (KAL),
      Dynein (DYN),
      Fukutin (FUK),
      Myosin IXA (MYIXA),
      Kininogen 1,
      α2-HS Glycoprotein,
      Complement C6,
      Complement C7,

Complement C8,

APOH, Beta-2-glycoprotein 1,

Serpin family A member 1,

Transferrin,

α1-B Glycoprotein,

Apo AI,

Apo D,

Alpha-1-microglobulin/bikunin precursor,

Carboxypeptidase N subunit 2,

Serine/arginine repetitive matrix protein 3,

Complement C1s,

Complement C1r,

Complement C2,

Complement C3,

Complement C4,

Complement C5,

Complement factor B,

Haptoglobin,

α2-macroglobulin,

Alpha-1-antitrypsin,

Ceruloplasmin,

Serpin family A member 3,

Serpin family A member 5,

Serpin family A member 7,

Serpin family G member 1,

Serpin family D member 1,

Coagulation factor V,

Prothrombin,

Plasminogen,

Protein S,

Afamin,

Transthyretin,

Hemoglobin subunit alpha,

Fetuin B,

Insulin like growth factor binding protein,

Alpha-2-glycoprotein 1,

Paraoxonase 1,

Dipeptidyl peptidase 4,

C-type lectin domain family 3 member B,

Galectin 3 binding protein,

Apolipoprotein AIV (Apo AIV),

Apolipoprotein CI (Apo CI),

Apolipoprotein CII (Apo CII),

Apolipoprotein E (Apo E),

Apolipoprotein M (Apo M),

Apolipoprotein B (Apo B),

Myosin IB,

Myosin XVIIIB,

Kinesin-like protein,

CD109 molecule,

Maltase-glucoamylase,

Sacsin molecular chaperone,

Dispatched RND transporter family member 2,

DNA Polymerase,

Trafficking protein particle complex subunit 2,

RB associated KRAB zinc finger,

Ciliogenesis-associated TTC17-interacting protein,

Proteasome subunit alpha type,

Regulating synaptic membrane exocytosis 2,
Extracellular matrix protein 1,
Cadherin 5,
Coiled-coil domain containing 178, and
Attractin,

said *at least two proteins* being preferably selected from

Collagen type XIII (COLXIII),
Hyaluronan-Binding Protein 2 (HABP2),
C4 Binding Protein Alpha (C4BP),
Complement Factor H (CFH),
Complement Factor I (CFI),
Fibrinogen,
Lumican (LUM),
Formin 1 (FMN),
Vitronectin (VTN),
Gelsolin (GS),
Angiotensinogen (AGT),
Adiponectin (ADP),
Transforming growth factor-$\beta$1 (TGF-$\beta$1),
Carboxyterminal Propeptid of Type I Procollagen (PICP),
C Reactive Protein (CRP),
$\alpha$-1-Acid Glycoprotein (alAGP),
Coagulationfactor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich $\alpha$-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK), and
Myosin IXA (MYIXA),

for use as biomarker in a method for the pre-clinical and/or early-stage detection and/or diagnosis of a kidney disease in a mammal.

6. The combination for use according to claim 5, wherein

ADP and PICP, or
AGT and GS, or
AGT and C9, or
GS and C9, or
GS and VTN, or
AGT and VTN, or
CFI and a1AGP, or
LRGP1 and C1q, or
CFH and C9, or
CRP and C1q

are determined.

7. The combination for use according to any one of claims 1 to 6, wherein the kidney disease is selected from chronic progressive kidney diseases

starting with glomerular injury (glomerulopathy),
preferably post-infectious glomerulopathy, IgA-nephropathy, Henoch-Schoenlein-Purpura, Focal segmental glomerulosclerosis, Alport syndrome, thin basement membrane nephropathy, benign familiar hematuria, or
starting on other parts of the body, with other dysregulations, and/or other parts of the kidney with implication on the glomerulus (nephropathy),
preferably metabolic syndrome, hypertension, heart failure, nephrotoxic medication, adipositas, diabetes mellitus (diabetic nephropathy), renal agenesis, renal hypoplasia, multicystic renal dysplasia, autosomal dominant polycystic disease, autosomal recessive polycystic disease, anomalies of the urinary tract, duplex kidneys, nephronophthisis.

8. The combination for use according any one of claims 1 to 7, wherein the concentration of each of the proteins is determined in a sample of a mammal,
and said concentration is preferably compared to a control value.

9. The combination for use according to any one of claims 1 to 8, wherein the sample is a bodily fluid, preferably urine or blood, such as blood serum, blood plasma, whole blood,

wherein more preferably the sample is urine,
and/or wherein the mammal is a human, or an animal, such as a dog, cat, rabbit, guinea pig, hamster, cattle, pig, horse, sheep, donkey, goat, red deer, or camel, wherein preferably the mammal is a human.

10. A *method* for the pre-clinical and/or early-stage detection and/or diagnosis of a kidney disease, comprising

(a) determining the concentration of at least two proteins in a sample of a mammal, wherein said at least two proteins are selected from

Collagen type XIII (COLXIII),
Hyaluronan-Binding Protein 2 (HABP2),
C4 Binding Protein (C4BP),
Complement Factor H (CFH), and
Complement Factor I (CFI),

or

wherein said at least two proteins are selected from
one protein which selected from

Collagen type XIII (COLXIII),
Hyaluronan-Binding Protein 2 (HABP2),
C4 Binding Protein (C4BP),
Complement Factor H (CFH), and
Complement Factor I (CFI),

and at least one further protein which is selected from

Fibrinogen,
Lumican (LUM),
Formin 1 (FMN),
Vitronectin (VTN),
Gelsolin (GS),
Angiotensinogen (AGT),
Adiponectin (ADP),
Transforming growth factor-$\beta$1 (TGF-$\beta$1),
Carboxyterminal Propeptid of Type I Procollagen (PICP),

C Reactive Protein (CRP),
α-1-Acid Glycoprotein (alAGP),
Coagulationfactor XIII (CFXIII),
Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK),
Myosin IXA (MYIXA),
Kininogen 1,
α2-HS Glycoprotein,
Complement C6,
Complement C7,
Complement C8,
APOH, Beta-2-glycoprotein 1,
Serpin family A member 1,
Transferrin,
α1-B Glycoprotein,
Apo AI,
Apo D,
Alpha-1-microglobulin/bikunin precursor,
Carboxypeptidase N subunit 2, and
Serine/arginine repetitive matrix protein 3,

said at least one further protein being preferably selected from

Fibrinogen, preferably Fibrinogen γ-chain (FGG),
Lumican (LUM),
Formin 1 (FMN),
Vitronectin (VTN),
Gelsolin (GS),
Angiotensinogen (AGT),
Adiponectin (ADP),
Transforming growth factor-β1 (TGF-β1),
Carboxyterminal Propeptid of Type I Procollagen (PICP), and
C Reactive Protein (CRP),

(b) comparing each of the concentrations determined in step (a) with a control value,

wherein a deviation of each of the concentrations determined in step (a) from said control value is indicative of a pre-clinical and/or early-stage kidney disease in said mammal.

11. A *method* for the pre-clinical and/or early-stage detection and/or diagnosis of a kidney disease, comprising

(a) determining the concentration of at least two proteins in a sample of a mammal,

wherein said at least two proteins are selected from

Collagen type XIII (COLXIII),
Hyaluronan-Binding Protein 2 (HABP2),

C4 Binding Protein (C4BP),

Complement Factor H (CFH),

Complement Factor I (CFI),

Fibrinogen,

Lumican (LUM),

Formin 1 (FMN),

Vitronectin (VTN),

Gelsolin (GS),

Angiotensinogen (AGT),

Adiponectin (ADP),

Transforming growth factor-$\beta$1 (TGF-$\beta$1),

Carboxyterminal Propeptid of Type I Procollagen (PICP),

C Reactive Protein (CRP),

$\alpha$-1-Acid Glycoprotein (alAGP),

Coagulationfactor XIII (CFXIII),

Complement Component 1q (C1q),

Complement Component 9 (C9),

Fibronectin (FN),

Ficolin 1 (FIC),

Hemopexin (HPX),

Inter-alpha-trypsin inhibitor 4 (IATI4),

Leucine Rich $\alpha$-2-Glycoprotein 1 (LRGP1),

Retinol-Binding Protein 4 (RBP4),

Serum Amyloid A (SAA),

Talin 1 (Tal),

Kallikrein (KAL),

Dynein (DYN),

Fukutin (FUK),

Myosin IXA (MYIXA),

Kininogen 1,

$\alpha$2-HS Glycoprotein,

Complement C6,

Complement C7,

Complement C8,

APOH, Beta-2-glycoprotein 1,

Serpin family A member 1,

Transferrin,

$\alpha$1-B Glycoprotein,

Apo AI,

Apo D,

Alpha-1-microglobulin/bikunin precursor,

Carboxypeptidase N subunit 2,

Serine/arginine repetitive matrix protein 3,

Complement C1s,

Complement C1r,

Complement C2,

Complement C3,

Complement C4,

Complement C5,

Complement factor B,

Haptoglobin,

$\alpha$2-macroglobulin,

Alpha-1-antitrypsin,

Ceruloplasmin,

Serpin family A member 3,

Serpin family A member 5,

Serpin family A member 7,

Serpin family G member 1,

Serpin family D member 1,
Coagulation factor V,
Prothrombin,
Plasminogen,
Protein S,
Afamin,
Transthyretin,
Hemoglobin subunit alpha,
Fetuin B,
Insulin like growth factor binding protein,
Alpha-2-glycoprotein 1,
Paraoxonase 1,
Dipeptidyl peptidase 4,
C-type lectin domain family 3 member B,
Galectin 3 binding protein,
Apolipoprotein AIV (Apo AIV),
Apolipoprotein CI (Apo CI),
Apolipoprotein CII (Apo CII),
Apolipoprotein E (Apo E4),
Apolipoprotein M (Apo M),
Apolipoprotein B (Apo B),
Myosin IB,
Myosin XVIIIB,
Kinesin-like protein,
CD109 molecule,
Maltase-glucoamylase,
Sacsin molecular chaperone,
Dispatched RND transporter family member 2,
DNA Polymerase,
Trafficking protein particle complex subunit 2,
RB associated KRAB zinc finger,
Ciliogenesis-associated TTC17-interacting protein,
Proteasome subunit alpha type,
Regulating synaptic membrane exocytosis 2,
Extracellular matrix protein 1,
Cadherin 5,
Coiled-coil domain containing 178, and
Attractin,

wherein said at least two proteins are preferably selected from

Collagen type XIII (COLXIII),
Hyaluronan-Binding Protein 2 (HABP2),
C4 Binding Protein (C4BP),
Complement Factor H (CFH),
Complement Factor I (CFI),
Fibrinogen,
Lumican (LUM),
Formin 1 (FMN),
Vitronectin (VTN),
Gelsolin (GS),
Angiotensinogen (AGT),
Adiponectin (ADP),
Transforming growth factor-$\beta$1 (TGF-$\beta$1),
Carboxyterminal Propeptid of Type I Procollagen (PICP),
C Reactive Protein (CRP),
$\alpha$-1-Acid Glycoprotein (alAGP),
Coagulationfactor XIII (CFXIII),

Complement Component 1q (C1q),
Complement Component 9 (C9),
Fibronectin (FN),
Ficolin 1 (FIC),
Hemopexin (HPX),
Inter-alpha-trypsin inhibitor 4 (IATI4),
Leucine Rich α-2-Glycoprotein 1 (LRGP1),
Retinol-Binding Protein 4 (RBP4),
Serum Amyloid A (SAA),
Talin 1 (Tal),
Kallikrein (KAL),
Dynein (DYN),
Fukutin (FUK), and
Myosin IXA (MYIXA),

(b) comparing each of the concentrations determined in step (a) with a control value,

wherein a deviation of each of the concentrations determined in step (a) from said control value is indicative of a pre-clinical and/or early-stage kidney disease in said mammal.

12. The method of claim 10 or 11, wherein the kidney disease is selected from chronic progressive kidney diseases

starting with glomerular injury (glomerulopathy),
preferably post-infectious glomerulopathy, IgA-nephropathy, Henoch-Schoenlein-Purpura, Focal segmental glomerulosclerosis, Alport syndrome, thin basement membrane nephropathy, benign familiar hematuria, or
starting on other parts of the body, with other dysregulations, and/or other parts of the kidney with implication on the glomerulus (nephropathy),
preferably metabolic syndrome, hypertension, heart failure, nephrotoxic medication, adipositas, diabetes mellitus (diabetic nephropathy), renal agenesis, renal hypoplasia, multicystic renal dysplasia, autosomal dominant polycystic disease, autosomal recessive polycystic disease, anomalies of the urinary tract, duplex kidneys, nephronophthisis.

13. The method of any one of claims 10 to 12, wherein the sample is a bodily fluid, preferably urine or blood, such as blood serum, blood plasma, whole blood, wherein more preferably the sample is urine,
and/or wherein the mammal is a human, or an animal, such as a dog, cat, rabbit, guinea pig, hamster, cattle, pig, horse, sheep, donkey, goat, red deer, or camel, wherein preferably the mammal is a human.

14. The method of any one of claims 10 to 13, wherein the concentrations of

COLXIII and C4BP, or
COLXIII and C4BP and HABP2, or
CFH and CFI are determined, or

wherein the concentrations of COLXIII, HABP2, C4BP, CFH, and CFI are determined.

15. The method of any one of claims 10 to 14, wherein the concentrations of

C4BP and ADP, or
COLXIII and FMN, or
HABP2 and TGF-β1, or
HABP2 and FGG, or
CFH and GS, or
CFI and GS, or
CFI and VTN, or
CFH and FGG, or
CFI and FGG are determined, or

wherein the concentrations of

ADP and PICP, or
AGT and GS, or
AGT and C9, or
GS and C9, or
GS and VTN, or
AGT and VTN, or
CFI and a1AGP, or
LRGP1 and C1q, or
CFH and C9, or
CRP and C1q are determined.

16. The method of any one of claims 10 to 15, wherein the control value is determined for each protein from healthy subjects and wherein the control value is preferably a standard concentration range,

and/or wherein a deviation of the concentration determined in step (a) from the median of the standard concentration range is indicative of a pre-clinical and/or early-stage kidney disease, preferably

a deviation higher than or equal to (>_) 2 times the median of the standard concentration range is indicative of a pre-clinical and/or early-stage kidney disease; or
a deviation lower than or equal to (≤) 0.5 times the median of the standard concentration range is indicative of a pre-clinical and/or early-stage kidney disease,

more preferably
a deviation higher than or equal to (>_) 2 times the median of the standard concentration range is indicative of a pre-clinical and/or early-stage kidney disease.

**Figure 1**

EP 4 397 976 A1

Figure 2

64

**Figure 3**

Europäisches
Patentamt

European
Patent Office

Office européen
des brevets

# EUROPEAN SEARCH REPORT

**Application Number**

**EP 23 15 0706**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GOMES ANA MARTA ET AL: "Potential Renal Damage Biomarkers in Alport Syndrome-A Review of the Literature", INTERNATIONAL JOURNAL OF MOLECULAR SCIENCES, vol. 23, no. 13, 30 June 2002 (2002-06-30) , page 7276, XP93045530, DOI: 10.3390/ijms23137276 Retrieved from the Internet: URL:https://www.ncbi.nlm.nih.gov/pmc/artic les/PMC9266446/pdf/ijms-23-07276.pdf> | 1-9 | INV. G01N33/68 |
| Y | * the whole document * * abstract * * table 1 * | 10-16 | |
| X | MIZDRAK MAJA ET AL: "Emerging Biomarkers for Early Detection of Chronic Kidney Disease", JOURNAL OF PERSONALIZED MEDICINE, vol. 12, no. 4, 31 March 2022 (2022-03-31) , page 548, XP93045531, DOI: 10.3390/jpm12040548 Retrieved from the Internet: URL:https://www.ncbi.nlm.nih.gov/pmc/artic les/PMC9025702/pdf/jpm-12-00548.pdf> | 1-9 | TECHNICAL FIELDS SEARCHED (IPC) G01N |
| Y | * the whole document * * abstract * * table 1 * * page 9, paragraph 2 * | 10-16 | |

-/--

The present search report has been drawn up for all claims

| Place of search | Date of completion of the search | Examiner |
|---|---|---|
| The Hague | 1 August 2023 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

**EUROPEAN SEARCH REPORT**

Europäisches Patentamt
European Patent Office
Office européen des brevets

Application Number

EP 23 15 0706

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KUMAR VIVEK ET AL: "Biomarkers for early diagnosis of diabetic kidney disease: still a long way to go", INDIAN JOURNAL OF MEDICAL RESEARCH, vol. 156, no. 1, 1 January 2022 (2022-01-01), page 14, XP93045535, IN ISSN: 0971-5916, DOI: 10.4103/ijmr.ijmr_1094_22 | 1-9 | |
| Y | * the whole document * | 10-16 | |
| X | US 2011/118127 A1 (RYU JAE-CHUN [KR] ET AL) 19 May 2011 (2011-05-19) | 1-9 | |
| Y | * the whole document * <br> * paragraphs [0031] - [0034] * | 10-16 | |
| X | WO 2018/094021 A1 (THE RES INSTITUTE AT NATIONWIDE CHILDRENS HOSPITAL [US]) 24 May 2018 (2018-05-24) <br> * the whole document * <br> * table 2 * | 1-16 | TECHNICAL FIELDS SEARCHED (IPC) |
| X | WÖRN MATTHIAS ET AL: "Proteasuria in nephrotic syndrome-quantification and proteomic profiling", JOURNAL OF PROTEOMICS, ELSEVIER, AMSTERDAM, NL, vol. 230, 12 September 2020 (2020-09-12), XP086337722, ISSN: 1874-3919, DOI: 10.1016/J.JPROT.2020.103981 [retrieved on 2020-09-12] <br> * the whole document * <br> * table 2 * | 1-16 | |

-/--

The present search report has been drawn up for all claims

| Place of search | Date of completion of the search | Examiner |
|---|---|---|
| The Hague | 1 August 2023 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

# EUROPEAN SEARCH REPORT

**Application Number**

EP 23 15 0706

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COLOMBO MARCO ET AL: "Biomarker panels associated with progression of renal disease in type 1 diabetes", DIABETOLOGIA, SPRINGER BERLIN HEIDELBERG, BERLIN/HEIDELBERG, vol. 62, no. 9, 20 June 2019 (2019-06-20), pages 1616-1627, XP036851035, ISSN: 0012-186X, DOI: 10.1007/S00125-019-4915-0 [retrieved on 2019-06-20] * the whole document * * table 2 * ----- | 1-16 | |
| X | EP 1 905 846 A2 (ELECTROPHORETICS LTD [GB]) 2 April 2008 (2008-04-02) * the whole document * * claims 1-24, particularly claims 9 and 18 * ----- | 1-16 | |
| X | WO 2022/189811 A1 (UNIV BRISTOL [GB]; SYNCONA INVESTMENT MAN LIMITED [GB] ET AL.) 15 September 2022 (2022-09-15) * the whole document * * the section bridging pages 37-38 * ----- | 1-16 | **TECHNICAL FIELDS SEARCHED (IPC)** |
| X | US 2012/129265 A1 (LUNDIN ULRIKA [AT] ET AL) 24 May 2012 (2012-05-24) * the whole document * * paragraph [0019] * ----- | 1-16 | |

The present search report has been drawn up for all claims

| Place of search | Date of completion of the search | Examiner |
|---|---|---|
| The Hague | 1 August 2023 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

**EP 23 15 0706**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

**1-16(partially)**

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 23 15 0706

The Search Division considers that the present European patent application does not comply with the requirements of unity of invention and relates to several inventions or groups of inventions, namely:

```
1. claims: 1-16(partially)

   A combination of biomarkers wherein at least one biomarker
   is COLXIIII.
                     ---


2-100. claims: 1-16(partially)

   A combination of biomarkers wherein at least one biomarker
   is HABP2, C4BP, CFH, CFI, fibrinogen, lumican etc (see list
   of claim 6).
                     ---
```

## ANNEX TO THE EUROPEAN SEARCH REPORT
## ON EUROPEAN PATENT APPLICATION NO.

EP 23 15 0706

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2023

| Patent document cited in search report | | Publication date | Patent family member(s) | | Publication date |
|---|---|---|---|---|---|
| US 2011118127 | A1 | 19-05-2011 | KR 20110053612 | A | 24-05-2011 |
| | | | US 2011118127 | A1 | 19-05-2011 |
| WO 2018094021 | A1 | 24-05-2018 | NONE | | |
| EP 1905846 | A2 | 02-04-2008 | AT 533859 | T | 15-12-2011 |
| | | | CA 2600168 | A1 | 05-03-2008 |
| | | | EP 1905846 | A2 | 02-04-2008 |
| | | | EP 2333116 | A2 | 15-06-2011 |
| | | | ES 2379430 | T3 | 26-04-2012 |
| | | | US 2008153092 | A1 | 26-06-2008 |
| | | | US 2012065095 | A1 | 15-03-2012 |
| WO 2022189811 | A1 | 15-09-2022 | NONE | | |
| US 2012129265 | A1 | 24-05-2012 | AU 2009347448 | A1 | 12-01-2012 |
| | | | BR PI0924639 | A2 | 23-02-2021 |
| | | | CA 2763948 | A1 | 09-12-2010 |
| | | | CN 102460160 | A | 16-05-2012 |
| | | | DK 2438441 | T3 | 18-08-2014 |
| | | | EP 2438441 | A1 | 11-04-2012 |
| | | | ES 2499415 | T3 | 29-09-2014 |
| | | | IL 216720 | A | 31-05-2015 |
| | | | JP 2012529015 | A | 15-11-2012 |
| | | | RU 2011153778 | A | 20-07-2013 |
| | | | SG 176655 | A1 | 30-01-2012 |
| | | | US 2012129265 | A1 | 24-05-2012 |
| | | | WO 2010139341 | A1 | 09-12-2010 |
| | | | ZA 201108907 | B | 29-08-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BAUM, A. et al.** Searching biomarker candidates in serum using multidimensional native chromatography. II Method evaluation with Alport syndrome and severe inflammation. *Journal of chromatography. B, Analytical technologies in the biomedical and life sciences,* 2008, vol. 876, 31-40
- **I. H. DE BOER ; M. L. CARAMORI ; J. C. N. CHAN ; H. J. L. HEERSPINK ; C. HURST ; K. KHUNTI et al.** Executive summary of the 2020 KDIGO Diabetes Management in CKD Guideline: evidence-based advances in monitoring and treatment. *Kidney Int,* 2020, vol. 98 (4), 839-848
- **DUFEK, B. et al.** Endothelin A receptor activation on mesangial cells initiates Alport glomerular disease. *Kidney international,* 2016, vol. 90, 300-310
- **GESSEL, M.M. et al.** Two Specific Sulfatide Species Are Dysregulated during Renal Development in a Mouse Model of Alport Syndrome. *Lipids,* 2019, vol. 54, 411-418
- **GOKA, S. ; COPELOVITCH, L. ; LEVY EREZ, D.** Long-term outcome among females with Alport syndrome from a single pediatric center. *Pediatric nephrology,* 2021, vol. 36, 945-951
- **GRODECKI, K.M. et al.** Treatment of X-linked hereditary nephritis in Samoyed dogs with angiotensin converting enzyme (ACE) inhibitor. *Journal of comparative pathology,* 1997, vol. 117, 209-225
- **GROSS, O. et al.** Preemptive ramipril therapy delays renal failure and reduces renal fibrosis in COL4A3-knockout mice with Alport syndrome. *Kidney Int,* 2003, vol. 63, 438-446
- **GROSS, O. et al.** Safety and Efficacy of the ACE-Inhibitor Ramipril in Alport Syndrome: The Double-Blind, Randomized, Placebo-Controlled, Multicenter Phase III EARLY PROTECT Alport Trial in Pediatric Patients. *ISRN pediatrics,* 2012, vol. 2012, 436046
- **GROSS, O. et al.** Advances and unmet needs in genetic, basic and clinical science in Alport syndrome: report from the 2015 International Workshop on Alport Syndrome. Nephrology, dialysis, transplantation: official publication of the European Dialysis and Transplant Association - European Renal Association, 2017, vol. 32, 916-924
- **GUO, J. et al.** Dysregulated Expression of microRNA-21 and Disease-Related Genes in Human Patients and in a Mouse Model of Alport Syndrome. *Hum Gene Ther,* 2019, vol. 30, 865-881
- **HERTZ, J.M. ; THOMASSEN, M. ; STOREY, H. ; FLINTER, F.** Clinical utility gene card for: Alport syndrome. *Eur J Hum Genet,* 2012, vol. 20
- **HUDSON, B.G. ; TRYGGVASON, K. ; SUNDARA-MOORTHY, M. ; NEILSON, E.G.** Alport's syndrome, Goodpasture's syndrome, and type IV collagen. *N Engl J Med,* 2003, vol. 348, 2543-2556
- **JERUMS G ; PANAGIOTOPOLOUS S ; PREMA-RATNE E ; MACISAAC RJ.** Integrating albuminurea and GFR in the assessment of diabetic nephropathy. *Nat Rev Nephrol,* 2009, vol. 5 (7), 397-406
- **KASHTAN, C.E.** Animal models of Alport syndrome. Nephrology, dialysis, transplantation: official publication of the European Dialysis and Transplant Association - European Renal Association, 2002, vol. 17, 1359-1362
- **KASHTAN, C.E. et al.** Clinical practice recommendations for the treatment of Alport syndrome: a statement of the Alport Syndrome Research Collaborative. *Pediatr Nephrol,* 2013, vol. 28, 5-11
- **KOOPMAN, J.J.E. ; VAN ESSEN, M.F. ; RENNKE, H.G. ; DE VRIES, A.P.J. ; VAN KOOTEN, C.** Deposition of the Membrane Attack Complex in Healthy and Diseased Human Kidneys. *Front Immunol,* 2020, vol. 11, 599974
- **KROMEYER-HAUSCHILD, K. ; WABITSCH, M. ; KUNZE, D. ; GELLER, F. ; GEIβ, H. C. ; HESSE, V. ; HEBEBRAND, J.** *Monatsschr. Kinderheilk.,* 2001, vol. 149
- **LEES, G.E.** Kidney diseases caused by glomerular basement membrane type IV collagen defects in dogs. *J Vet Emerg Crit Care (San Antonio),* 2013, vol. 23, 184-193
- **MUCKOVA, P. et al.** Preclinical Alterations in the Serum of COL(IV)A3(-)/(-) Mice as Early Biomarkers of Alport Syndrome. *JProteome Res,* 2015, vol. 14, 5202-5214
- **NAGEL, M. ; NAGORKA, S. ; GROSS, O.** Novel COL4A5, COL4A4, and COL4A3 mutations in Alport syndrome. *Hum Mutat,* 2005, vol. 26, 60
- **NOONE, D. ; LICHT, C.** An update on the pathomechanisms and future therapies of Alport syndrome. *Pediatr Nephrol,* 2013, vol. 28, 1025-1036
- **POHL, M. et al.** Diagnosis of Alport syndrome--search for proteomic biomarkers in body fluids. *Pediatric nephrology,* 2013, vol. 28, 2117-2123

- **RHODE, H. ; MUCKOVA, P. ; BUCHLER, R. ; WENDLER, S. ; TAUTKUS, B. ; VOGEL, M. et al.** A next generation setup for pre-fractionation of non-denatured proteins reveals diverse albumin proteoforms each carrying several post-translational modifications. *Sci Rep,* 2019, vol. 9 (1), 11733
- **ROCCELLA EJ et al.** National High Blood Pressure Education Program Working Group on High Blood Pressure in Children and Adolescents). The fourth report on the diagnosis, evaluation, and treatment of high blood pressure in children and adolescents. *Pediatrics,* 2004, vol. 114 (S2), 555-576
- **SAVIGE, J et al.** Expert consensus guidelines for the genetic diagnosis of Alport syndrome. *Pediatr Nephrol,* 2018
- **SAVIGE, J. et al.** Expert consensus guidelines for the genetic diagnosis of Alport syndrome. *Pediatric nephrology,* 2019, vol. 34, 1175-1189
- **WENDLER, S. ; TAUTKUS, B. ; NEMITZ, S. ; PESEK, J. ; KRÜGER, T. ; OPITZ, S. ; BARTZ, M. ; RICHTER, S. ; OEHME, H. ; HAENEL, T.** Automations Systems of the 21st Century. nova publishers, 2013, 1-50
- **YAMAMURA, T. et al.** Development of an exon skipping therapy for X-linked Alport syndrome with truncating variants in COL4A5. *Nat Commun,* 2020, vol. 11, 2777